(19) Europäisches Patentamt
European Patent Office
Office européen des brevets

(11) **EP 4 265 172 A1**

(12) **EUROPEAN PATENT APPLICATION**
published in accordance with Art. 153(4) EPC

(43) Date of publication:
**25.10.2023 Bulletin 2023/43**

(21) Application number: **21910142.5**

(22) Date of filing: **29.11.2021**

(51) International Patent Classification (IPC):
***A61B 3/06*** (2006.01)

(52) Cooperative Patent Classification (CPC):
**A61B 3/06**

(86) International application number:
**PCT/JP2021/043584**

(87) International publication number:
**WO 2022/137990 (30.06.2022 Gazette 2022/26)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **21.12.2020 JP 2020211755**

(71) Applicant: **Nikon-Essilor Co., Ltd.
Tokyo 130-0026 (JP)**

(72) Inventor: **MONMA Tsukasa
Tokyo 130-0026 (JP)**

(74) Representative: **Hoffmann Eitle
Patent- und Rechtsanwälte PartmbB
Arabellastraße 30
81925 München (DE)**

(54) **VISUAL FUNCTION EXAMINATION DEVICE, SPECTACLE LENS PRESENTATION SYSTEM, PRINTED MATTER, VISUAL FUNCTION EXAMINATION METHOD, SPECTACLE LENS PRESENTATION METHOD, AND PROGRAM**

(57)    A visual function examination device includes a response information acquisition unit configured to acquire response information indicating a response about glare felt by a subject to a visual target based on a glare illusion and a glare index calculation unit configured to calculate an index for the glare felt by the subject on the basis of the response information acquired by the response information acquisition unit.

FIG. 4

EP 4 265 172 A1

**Description**

[Technical Field]

The present invention relates to a visual function examination device, a spectacle lens presentation system, a printed matter, a visual function examination method, a spectacle lens presentation method, and a program.
Priority is claimed on Japanese Patent Application No. 2020-211755, filed December 21, 2020, the content of which is incorporated herein by reference.

[Background Art]

It is necessary to be able to examine a subject's sensitivity to glare in order to provide spectacle lenses suitable for the subject. For example, an evaluation device for evaluating the glare of a light source felt by a subject is known (Patent Document 1).

[Citation List]

[Patent Document]

[Patent Document 1]
Japanese Unexamined Patent Application, First Publication No. 2008-93131

[Summary of Invention]

According to an aspect of the present invention, there is provided a visual function examination device including: a response information acquisition unit configured to acquire response information indicating a response about glare felt by a subject to a visual target based on a glare illusion; and a glare index calculation unit configured to calculate an index for the glare felt by the subject on the basis of the response information acquired by the response information acquisition unit.
According to an aspect of the present invention, there is provided a spectacle lens presentation system including: the above-described visual function examination device; and a spectacle lens presentation device, wherein the spectacle lens presentation device includes a spectacle lens decision unit configured to decide on spectacle lenses suitable for the subject on the basis of the index calculated by the visual function examination device; and a spectacle lens information presentation unit configured to present information about the spectacle lenses decided on by the spectacle lens decision unit.
According to an aspect of the present invention, there are provided a plurality of printed matters on which a plurality of visual targets, which are based on a glare illusion and cause the glare illusion to different degrees, are printed.
According to an aspect of the present invention, there is provided a visual function examination method including: acquiring response information indicating a response about glare felt by a subject to a visual target based on a glare illusion; and calculating an index for the glare felt by the subject on the basis of the acquired response information.
According to an aspect of the present invention, there is provided a spectacle lens presentation method including: acquiring response information indicating a response about glare felt by a subject to a visual target based on a glare illusion; calculating an index for the glare felt by the subject on the basis of the acquired response information; deciding on spectacle lenses suitable for the subject on the basis of the calculated index; and presenting information about the decided spectacle lenses.
According to an aspect of the present invention, there is provided a program for causing a computer to execute: a response information acquisition step of acquiring response information indicating a response about glare felt by a subject to a visual target based on a glare illusion; and a glare index calculation step of calculating an index for the glare felt by the subject on the basis of the response information acquired in the response information acquisition step.

[Brief Description of Drawings]

FIG. 1 is a diagram showing an example of a configuration of a visual function examination system according to a first embodiment.
FIG. 2 is a diagram showing an example of a pattern in which a glare illusion is caused according to the first embodiment.

FIG. 3 is a diagram showing an example of a hardware configuration of a visual function examination device according to the first embodiment.

FIG. 4 is a diagram showing an example of a functional configuration of the visual function examination system according to the first embodiment.

FIG. 5 is a diagram showing an example of a glaring target according to the first embodiment.

FIG. 6 is a diagram showing an example of parameters for creating a glare illusion image according to the first embodiment.

FIG. 7 is a diagram showing an example of parameters used for creating a glare illusion image according to the first embodiment.

FIG. 8 is a diagram showing an example of an overview of a double staircase method according to the first embodiment.

FIG. 9 is a diagram showing an example of a relationship between the number of trials in the double staircase method and a luminance value of a center region of a presented glaring target according to the first embodiment.

FIG. 10 is a diagram showing an example of luminance values of regions of the glare illusion image for use in the double staircase method according to the first embodiment.

FIG. 11 is a diagram showing another example of a glare illusion image according to the first embodiment.

FIG. 12 is a diagram showing an example of luminance values in regions of the glare illusion image for use in the double staircase method according to the first embodiment.

FIG. 13 shows an example of luminance values in regions for another glare illusion image for use in the double staircase method according to the first embodiment.

FIG. 14 is a diagram showing an example of an overview of a magnitude estimation method according to the first embodiment.

FIG. 15 is a diagram showing an example of an examination result in the magnitude estimation method according to the first embodiment.

FIG. 16 is a diagram showing an example of a graph in which an average evaluation value for a relative value of luminance of a center region of the glaring target on the basis of the examination result of the magnitude estimation method is plotted according to the first embodiment.

FIG. 17 is a diagram showing an example of a graph of a case where graphs in which an average evaluation value for a relative value of luminance of a center region of the glaring target is plotted on the basis of the examination result of the magnitude estimation method are plotted in both logarithms according to the first embodiment.

FIG. 18 is a diagram showing an example of an index calculation process according to the first embodiment.

FIG. 19 is a diagram showing an example of a mechanical configuration of the visual function examination system according to a modified example of the first embodiment.

FIG. 20 is a diagram showing an example of a functional configuration of a visual function examination system according to a second embodiment.

FIG. 21 is a diagram showing an example of a functional configuration of a visual function examination system according to a third embodiment.

FIG. 22 is a diagram showing an example of a functional configuration of a visual function examination system according to a fourth embodiment.

FIG. 23 is a diagram showing an example of a spectacle lens wearing image according to the fourth embodiment.

FIG. 24 is a diagram showing an example of average luminance of a glaring target included in the spectacle lens wearing image when a transmittance cut ratio is variously changed according to the fourth embodiment.

FIG. 25 is a diagram showing an example of average luminance of a glaring target included in the spectacle lens wearing image when the transmittance cut ratio is variously changed according to the fourth embodiment.

FIG. 26 is a diagram showing an example of a configuration of a visual function examination system 1g according to a fifth embodiment.

[Description of Embodiments]

(First embodiment)

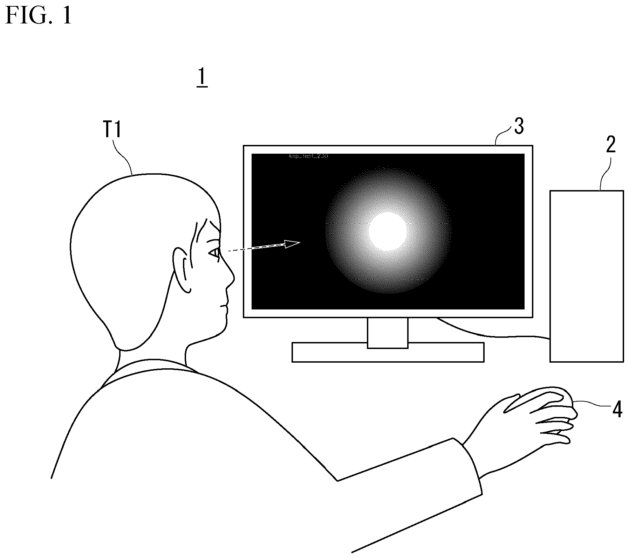

Hereinafter, a first embodiment will be described in detail with reference to the drawings. FIG. 1 is a diagram showing an example of a configuration of a visual function examination system 1 according to the present embodiment. The visual function examination system 1 is a system for examining the sensitivity of a subject T1 to glare as a visual function of the subject T1. The visual function examination system 1 examines the sensitivity of the subject T1 to glare using a glare illusion. The glare illusion is a type of psychological visual illusion in humans who perceive light.

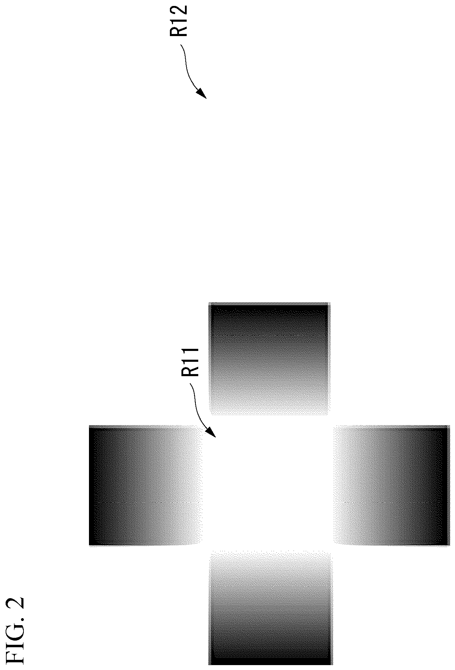

Here, the glare illusion will be described with reference to FIG. 2. FIG. 2 is a diagram showing an example of a pattern in which a glare illusion is caused according to the present embodiment. In FIG. 2, a white portion R11 is surrounded by rectangles each having a luminance gradient on white test paper. The rectangle has a gradient in which the luminance increases from the periphery far from the white portion R11 to the center near the white portion R11. The luminance of the white portion R11 and the luminance of a background R12 are the same as each other. The white portion R11 is perceived as psychologically brighter than the background R12 because it is surrounded by rectangular fragments having the luminance gradient described above, and the impression that the white portion R11 is luminous is created. The glare illusion is such a visual illusion phenomenon.

The glare illusion is caused by staring at a region (called a center region) surrounded by a region with a luminance gradient (called a peripheral region). In the glare illusion, a case where the luminance gradient of the peripheral region induces the impression of luminescence is conceivable. Thus, a degree of impression of luminescence in the center region can be changed by changing the luminance gradient of the peripheral region.

The visual function examination system 1 includes a visual function examination device 2, a display device 3, and an input device 4. The display device 3 displays a glare illusion image A1. The glare illusion image A1 is an image including a glaring target G1. The glaring target G1 is a visual target based on the glare illusion. By operating the input device 4, the subject inputs a response to the visual function examination device 2 as to whether or not he or she has felt glare when staring at the glare illusion image A1 displayed on the display device 3. The visual function examination device 2 calculates an index for the glare felt by the subject T1 based on the response.

Also, the visual function examination device 2 includes a function of generating the glare illusion image A1 based on the input parameter A4.

Details of the glare illusion image A1 and the parameter A4 will be described below.

As an example, the visual function examination device 2 is a personal computer (PC). As an example, the visual function examination device 2 is provided in an optician shop, an ophthalmic clinic, or the like.

The display device 3 is, for example, a liquid crystal display, an organic electroluminescence (EL) display, or the like. In addition to a display device that requires a backlight such as a liquid crystal display, the display device 3 may be a self-luminous display device or a projection type display device.

The input device 4 is a mouse as an example. The input device 4 may be a keyboard or a button switch.

Also, the input device 4 may be a touch panel configured to be integrated with the display device 3.

Also, the visual function examination device 2 may be a smartphone or a tablet terminal. When the visual function examination device 2 is a smartphone or a tablet terminal, the display device 3 and the input device 4 are provided to be integrated with the visual function examination device 2 as a touch panel. The display device 3 may be a head-mounted display.

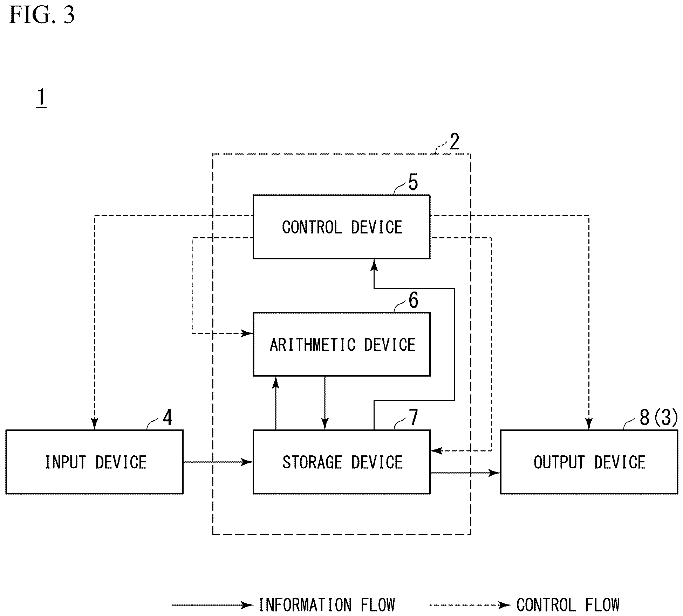

FIG. 3 is a diagram showing an example of a hardware configuration of the visual function examination device 2 according to the present embodiment. The visual function examination device 2 includes a control device 5, an arithmetic device 6, and a storage device 7.

The control device 5 reads a program from the storage device 7 and executes various types of control according to the read program. The arithmetic device 6 performs various types of arithmetic operations. The control device 5 and the arithmetic device 6 constitute a central processing unit (CPU).

The storage device 7 includes a main storage device and an auxiliary storage device. The main storage unit temporarily stores programs and data. The main storage device is a so-called memory, which is a volatile storage device. The auxiliary storage device is a large-capacity and non-volatile storage device. The auxiliary storage device is configured using a storage device such as a magnetic hard disk device or a semiconductor storage device such as a flash memory and an optical storage device such as a CD or a DVD.

The output device 8 outputs various types of information. The output device 8 includes the display device 3 shown in FIG. 1. The output device 8 may include a printer. The printer is, for example, a printer using a method of a laser printer, an inkjet printer, a thermal transfer printer, or the like. The printer performs one or more of a color printing process and a black and white printing process.

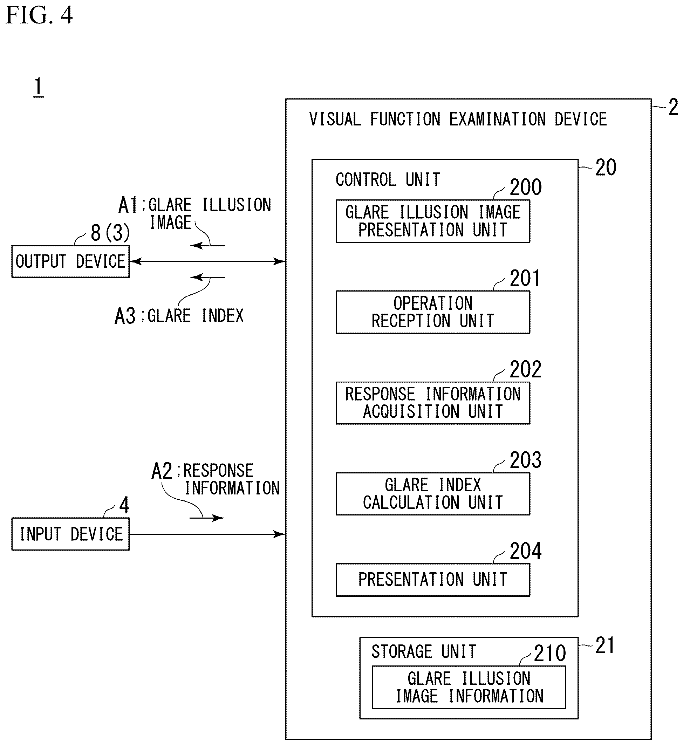

Next, a functional configuration of the visual function examination system 1 will be described with reference to FIG. 4. FIG. 4 is a diagram showing an example of the functional configuration of the visual function examination system 1 according to the present embodiment. The visual function examination device 2 includes a control unit 20 and a storage unit 21. The control unit 20 includes a glare illusion image presentation unit 200, an operation reception unit 201, a response information acquisition unit 202, a glare index calculation unit 203, and a presentation unit 204. The control unit 20 is implemented by the control device 5 and the arithmetic device 6 shown in FIG. 3. Each functional unit provided in the control unit 20 is implemented by the control device 5 reading a program from the storage unit 21 and executing a process.

The glare illusion image presentation unit 200 presents the glare illusion image A1. Here, the glare illusion image presentation unit 200 presents the glare illusion image A1 to the subject T1 by displaying the glare illusion image A1 on the display device 3.

The operation reception unit 201 receives various types of operations from the input device 4. The various types of operations received by the operation reception unit 201 include an operation in which the subject T1 inputs a response from the input device 4. Here, the response input by the subject T1 is a response about the glare felt by the subject T1 with respect to the glare illusion image A1 presented by the glare illusion image presentation unit 200.

The response information acquisition unit 202 acquires a response from the subject T1 input through the operation received by the operation reception unit 201 as response information A2. The response information A2 is information indicating a response about glare felt by the subject T1 with respect to the glaring target G1.

The glare index calculation unit 203 calculates a glare index A3 on the basis of the response information A2 acquired by the response information acquisition unit 202. The glare index A3 is an index for the glare felt by the subject T1.

The presentation unit 204 presents the glare index A3 calculated by the glare index calculation unit 203. The presentation unit 204 presents the glare index A3 by, for example, causing the glare index A3 to be displayed on the display device 3.

The storage unit 21 stores various types of information. The various types of information stored by the storage unit 21 include glare illusion image information 210. The glare illusion image information 210 is information indicating the glare illusion image A1. The storage unit 21 is implemented by an auxiliary storage device included in the storage device 7 shown in FIG. 3.

[Glare illusion image]

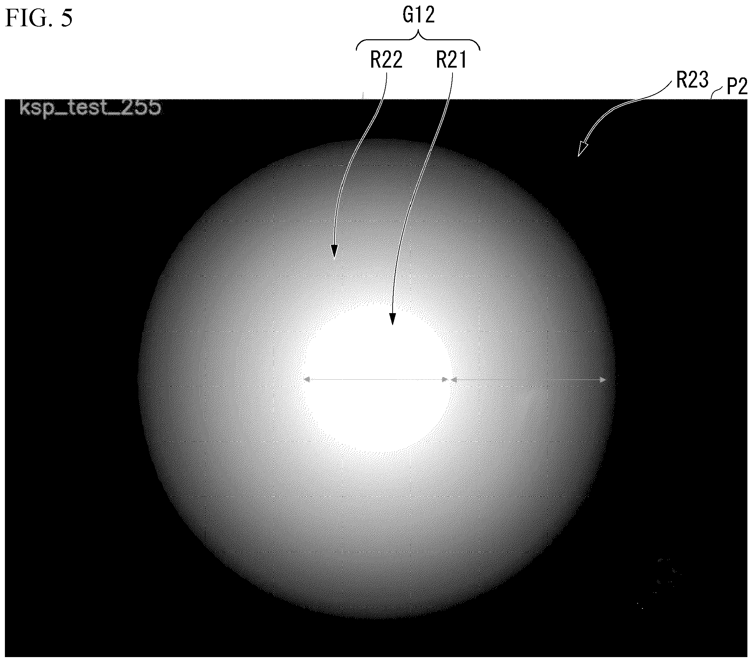

The glaring target G1 will be described with reference to FIG. 5. FIG. 5 is a diagram showing an example of a glaring target G12 according to the present embodiment. In FIG. 5, the glaring target G12 is included in a glare illusion image P2, which is an example of the glare illusion image A 1. In the present embodiment, the glare illusion image A1 is generated in advance and stored in the storage unit 21.

The glare illusion image P2 is a grayscale image as an example. The glare illusion image P2 is a rectangular image. In the glare illusion image P2, the glaring target G1 is drawn on a background R23. The glaring target G1 includes a center region R21 and an induction region R22. Therefore, the glare illusion image P2 includes the center region R21, the induction region R22, and the background R23. In the glare illusion image P2, the center region R21 is a circular region located at the center of the glare illusion image P2.

In the glare illusion image P2, the center region R21 is displayed at prescribed luminance. The induction region R22 is a shape surrounded by concentric circles consisting of two circles. In the induction region R22, an inner circle of the concentric circles coincides with the outline of the center region R21.

In the glare illusion image P2, the induction region R22 has a luminance gradient in which the luminance increases from the outside to the inside. The induction region R22 has maximum luminance at an innermost portion and the maximum luminance is equal to the luminance of the center region R21. The induction region R22 has minimum luminance in an outermost portion and the minimum luminance is equal to the luminance of the background R23.

The background R23 is the remaining portion other than the center region R21 and the induction region R22 of the glare illusion image P2. The background R23 is displayed at prescribed luminance less than the luminance of the center region R21.

Although an example in which the glare illusion image A1 is a grayscale image like the glare illusion image P2 shown in FIG. 5 will be described in the present embodiment, the present invention is not limited thereto. The glare illusion image A1 may be created and/or displayed using any color.

In this case, for example, in a consideration in an RGB color space, each value (R, G, or B) of the RGB values takes a certain value in the center region and the background. On the other hand, in the induction region, any one or more of values (R, G, and B) among the RGB values changes with a certain gradient.

Also, for example, when considered in an HSV color space, S (saturation) and H (lightness) take certain values in the center region and the background and a value of at least one of S (saturation) and H (lightness) in the center region is larger than that of the background. In the induction region, each value (S or H) changes with a certain gradient. H (hue) corresponds to the type of color and may have any value. In terms of the HSV color space, in the case of a grayscale image, only H (lightness) changes.

The subject T1 stares at the center region R21 in the examination of the sensitivity to glare. The center region R21 and the induction region R22 are in contact with each other in a portion where the luminance of the induction region R22 is maximum. Thereby, it is possible to increase a luminance amount of the center region R21 psychologically perceived by the subject T1 to an actual luminance amount or more. For example, by measuring the increased amount in the magnitude estimation method, it has been shown that the center region is perceived as 20% to 35% brighter than the referenced white color due to the glare illusion.

The glare effect will differ according to a color tone and shape of the luminance gradient provided in the induction region. The luminance gradient of luminance is, for example, a gradient that follows a positive part of a Gaussian function. The luminance gradient of the luminance may be changed linearly and then changed by performing conversion based on gamma correction. According to the luminance gradient provided in the induction region, a degree of a luminescence phenomenon in the center region can be changed. Also, it is possible to cause a stronger luminescence phenomenon in the center region by arranging streaks radially with respect to the generated glaring target. It is also possible to change the luminescence phenomenon of the center region by changing the maximum luminance and the minimum luminance of the induction region and changing the size and shape of the induction region.

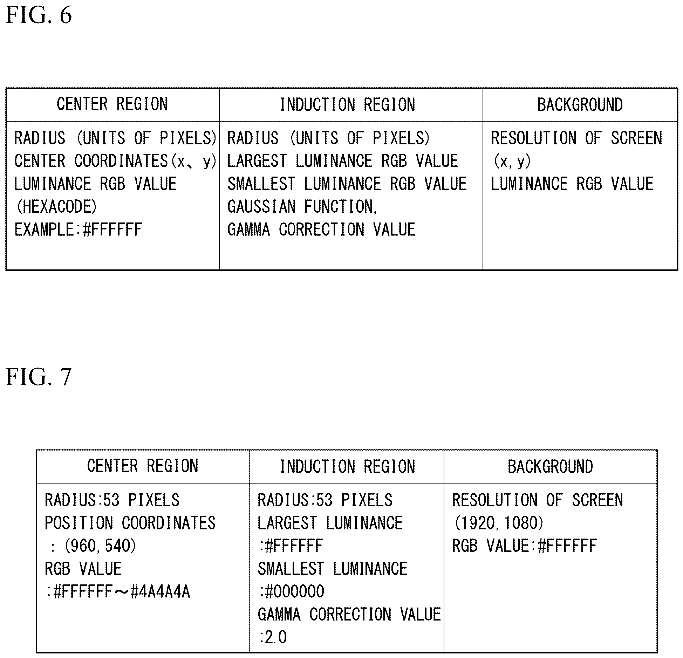

FIG. 6 shows an example of parameters for creating the glare illusion image A1. The parameters shown in FIG. 6 are parameters used in a case where the glare illusion image A1 is created using an open-source computer vision library OpenCV as an example. The center region R21 is designated by a radius, central coordinates, and luminance. The luminance is designated, for example, using an RGB value by hexacode. The induction region R22 is designated by a radius, maximum luminance, minimum luminance, and a luminance gradient. The background R23 is designated by screen resolution or luminance.

A glare illusion image P2 is an image created on the basis of grayscale. A diameter of the circular center region R21 is 26.288 mm (53 pixels). A diameter of an inner circle of the induction region R22, which is a shape of a concentric circle, is 26.288 mm (53 pixels). An outer shape of the background R23 is equal to a maximum display range of the display. When the glare illusion image P2 is displayed on a 21.5-inch display with a resolution of 1920×1080, the background R23 is displayed as a rectangle with a horizontal length of 476.6 mm and a vertical length of 268.1 mm.

FIG. 7 shows parameters used to create the glare illusion image P2. The parameters shown in FIG. 7 are parameters used when the glare illusion image A1 is created using OpenCV like the parameters shown in FIG. 6.

[Examination method]

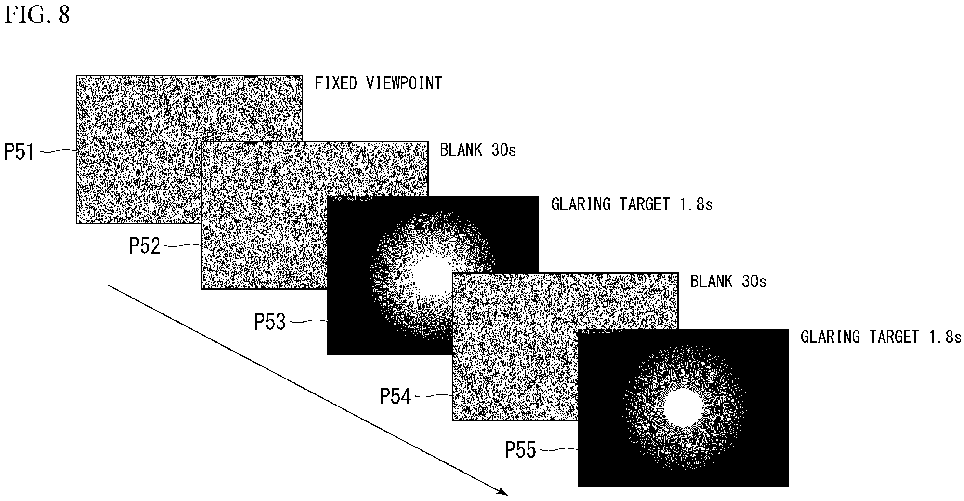

Next, a method of examining the sensitivity of the subject T1 to glare using the visual function examination system 1 will be described with reference to FIGS. 8 to 12. Various examination methods have been proposed from experimental psychology by quantifying a way humans feel and an amount of sensation to stimuli from the outside world. In the examination method of the visual function examination system 1, any examination method among these examination methods may be used.

In the present embodiment, as an example, an examination method of obtaining an absolute threshold in a double staircase method will be described. FIG. 8 is a diagram showing an example of an overview of the double staircase method according to the present embodiment. Here, the absolute threshold is a boundary between whether or not the subject T1 feels glare and corresponds to a psychological quantity when the subject T1 begins to feel glare. In the present embodiment, a physical quantity for the absolute threshold corresponding to the psychological quantity when glare begins to be felt is calculated.

In the examination method of the present embodiment, the subject T1 sits in a chair so that a distance between the display device 3 and the eyes of the subject T1 is 1.5 m. A fixed viewpoint image P51 is presented to the display device 3. A fixed viewpoint is presented in the fixed viewpoint image P51. The position of the fixed viewpoint presented in the fixed viewpoint image P51 is adjusted to match an eye height of the subject T1. The subject T1 stares at the glaring target G1 presented on the display device 3 and determines whether or not to feel glare. The subject T1 responds by pressing a button switch (an example of the input device 4) held in the hand when glare is felt.

First, the visual function examination device 2 presents a blank image P52 in which a blank is displayed for 30 sec. Subsequently, the visual function examination device 2 presents a glare illusion image P53 including a glaring target for 1.8 sec. After the glaring target is presented, a blank image P54 is presented for 30 sec. The subject T1 responds as to whether or not glare was felt during 30 sec when the blank image P54 is presented. When a period of 30 sec has elapsed, the visual function examination device 2 presents a glare illusion image P55 including a glaring target for 1.8 sec. Here, a luminance value of the center region of the glaring target G1 included in the glare illusion image P55 and a luminance value of the center region of the glare illusion image P53 presented above are different.

In the double staircase method of the examination method of the present embodiment, the glare illusion image and the blank image are alternately presented to the subject T1 while the luminance value of the center region is changed in a plurality of glare illusion images A1. The plurality of glare illusion images A1 include an ascending series in which the luminance value of the center region increases in the order in which they are presented and a descending series in which the luminance value of the center region decreases in the order in which they are presented. In the double staircase method, the glare illusion image A1 included in the ascending series and the glare illusion image A1 included in the descending series are alternately presented in the presented glare illusion images A1. That is, in the double staircase method, the ascending series and the descending series are presented in parallel. In the present embodiment, for each of the ascending series and the descending series, the luminance value of the center region of the glaring target changes in 15 steps. The glare illusion image P53 shown in FIG. 8 is a glare illusion image A1 having a largest luminance value of the center region in the descending series and the glare illusion image P55 is a glare illusion image A1 having a smallest luminance value of the center region in the ascending series.

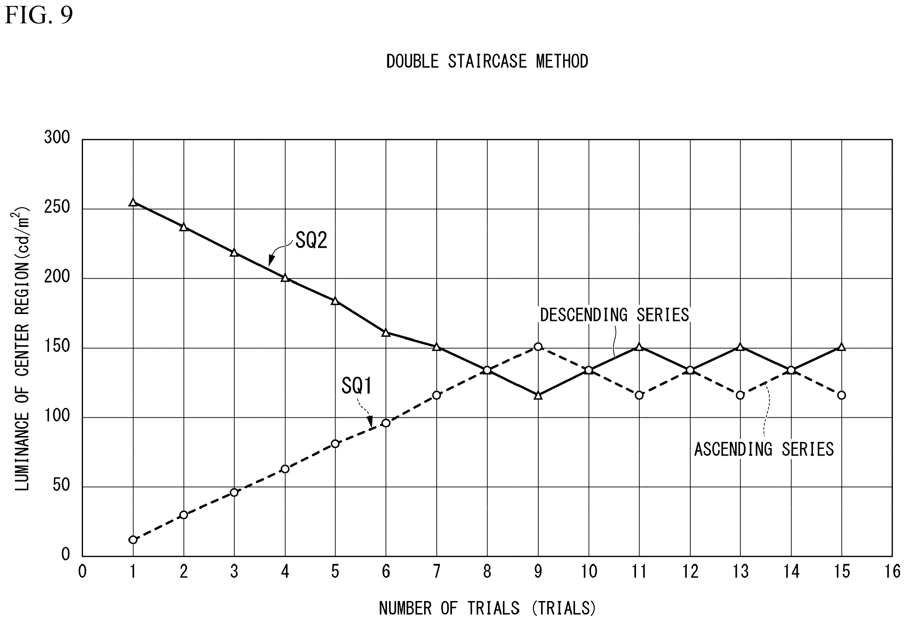

FIG. 9 is a diagram showing an example of a relationship between the number of trials and the luminance value of the center region of the presented glaring target in the double staircase method according to the present embodiment. In FIG. 9, an ascending series luminance value graph SQ1, which is a graph showing the ascending series luminance value for each number of trials, and a descending series luminance value graph SQ2, which is a graph showing the descending series luminance value for each number of trials, are shown.

When the subject T1 responds that glare has been felt with respect to the glare illusion image A1 included in the ascending series, a glare illusion image A1 obtained by decreasing the luminance value of the center region step by step from the glare illusion image A1 is presented at the time of the next and subsequent ascending series presentations in the ascending series. When the subject T1 responds that glare has not been felt in the next and subsequent ascending series, a glare illusion image A1 obtained by increasing the luminance value of the center region step by step from the glare illusion image A1 is presented at the time of the next and subsequent ascending series presentations in the ascending series. Subsequently, the luminance value is decreased when the response indicates that glare has been felt and the luminance value is increased when the response indicates that glare has not been felt, such that the glare illusion image A1 included in the ascending series is presented.

As in the ascending series, the glare illusion images A1 included in the descending series are presented in order so that the luminance value is decreased when the response indicates that glare has been felt and the luminance value is increased when the response indicates that glare has not been felt.

As described above, when the ascending series and the descending series are presented alternately, a luminance value difference between the center regions of the ascending series and the descending series decreases with the number of trials. Ultimately, the response indicates that glare has been felt and the response indicates that glare has not been felt are repeated so that the number of trials is shown in a range of "9" to "15" in FIG. 9. In the present embodiment, after the responses were repeated, the examination was completed at the trial number "15" of the seventh trial.

An average value between luminance values of the center region of each of the ascending series and the descending series in the range where the responses are repeated is calculated as an absolute threshold. From the calculated absolute threshold, the sensitivity to glare is confirmed as the sensitivity of the subject T1 to glare. According to a magnitude estimation method for use in the experiment of experimental psychology to be described below, a relationship between physical and psychological quantities can be represented by one equation. The calculated absolute threshold corresponds to a psychological quantity and appropriate lenses for the subject T1 can be proposed by estimating the physical quantity from the absolute threshold in the magnitude estimation method.

Also, the calculated absolute threshold is an example of the glare index A3 described above.

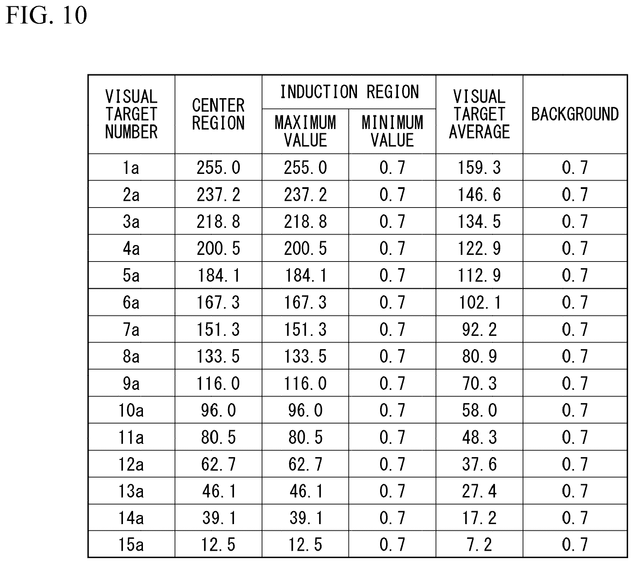

An example of luminance values in regions of the glare illusion image P2 for use in the double staircase method is shown in FIG. 10. As described above, in the present embodiment, the luminance value of the center region of the glaring target changes in 15 steps for each of the ascending series and the descending series. Glaring targets from a visual target number "15a" to a target number "1a" constitute the ascending series in that order. Glaring targets from the visual target number "1a" to the visual target number "15a" constitute the descending series in that order. As shown in FIG. 10, the maximum luminance of the induction region is equal to the luminance value of the center region and changes with the visual target number. The minimum luminance is constant for the visual target number. The background luminance value is the same as the minimum luminance of the induction region. The visual target average is an average of the luminance values of the center region R21 and the induction region R22. Also, the unit of the luminance value is $cd/m^2$.

The glare illusion image A1 for use in the double staircase method is not limited to the glare illusion image P2 shown in FIG. 5.

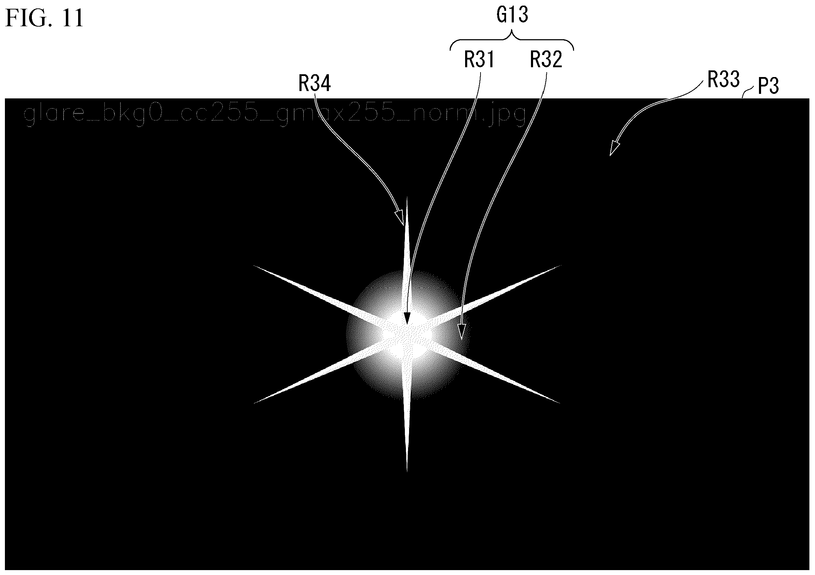

FIG. 11 is a diagram showing another example of the glare illusion image A1 according to the present embodiment. In FIG. 11, the glare illusion image P3 is shown as the glare illusion image A1. The glare illusion image P3 includes a center region R31, an induction region R32, and a background R33 like the glare illusion image P2. The center region R31 and the induction region R32 form a glaring target G13. In the glare illusion image P3, a light streak R34 having a gradation extending in a radial direction from the center region R31 is added in the glare illusion image A1 like the glare illusion image P2. The glare illusion image P3 includes six light streaks R34 extending in different directions by 60 degrees.

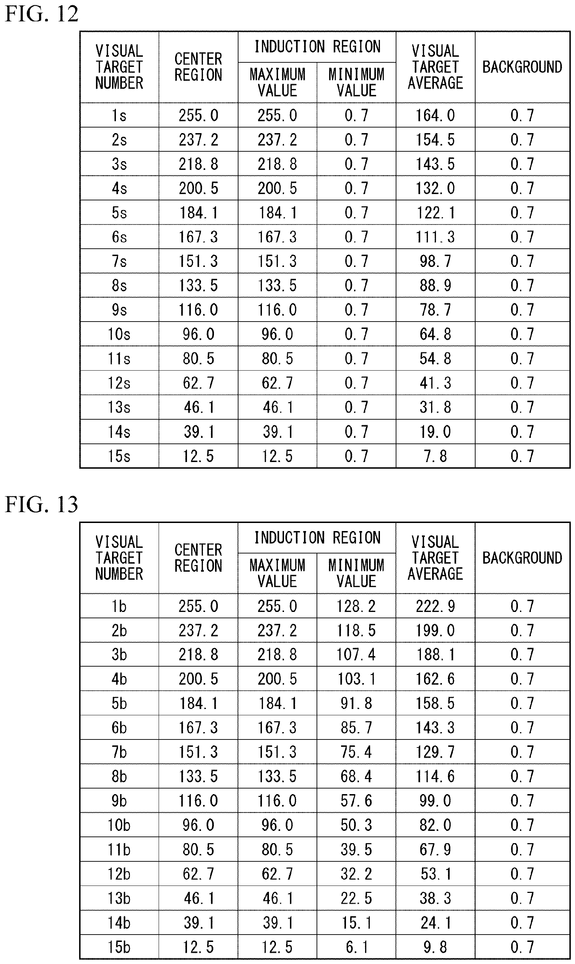

FIG. 12 is a diagram showing an example of luminance values of regions of the glare illusion image P3 for use in the double staircase method according to the present embodiment. The average luminance of the visual target is slightly higher than the average luminance of the visual target shown in FIG. 10 when the glare illusion image A1 like the glare illusion image P2 is used.

In FIG. 13, an example of luminance values in regions for another glare illusion image for use in the double staircase method according to the present embodiment is shown. In the example shown in FIG. 13, the induction region has a luminance gradient at which luminance decreases as it moves toward an outer boundary. In the example shown in FIG. 13, compared with the examples shown in FIGS. 10 and 12, the minimum value of the luminance value of the induction region is half the maximum value and the luminance gradient changes gradually.

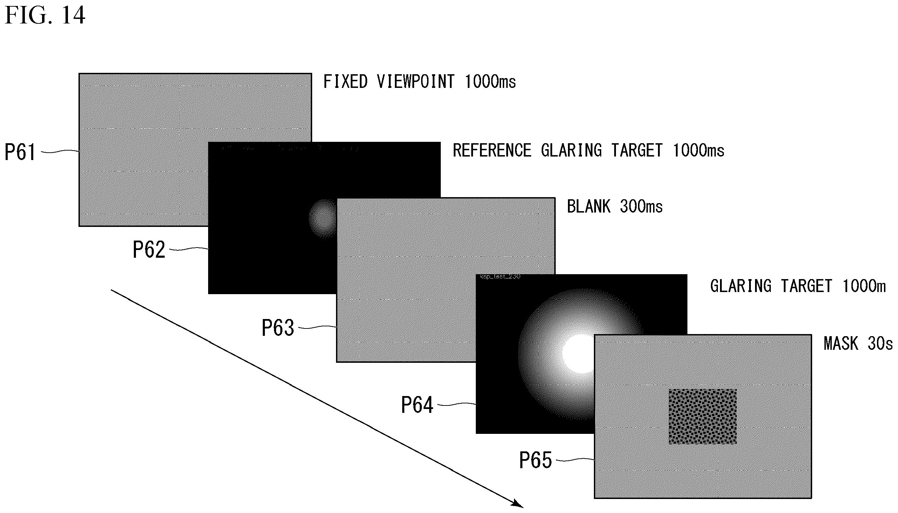

Here, a magnitude estimation method will be described as an example of an examination method other than the double staircase method with reference to FIG. 14. FIG. 14 is a diagram showing an example of an overview of the magnitude estimation method according to the present embodiment. In the magnitude estimation method, physical quantities and psychological quantities can be shown according to the following Eq. (1).
[Math. 1]

$$J = kI^p \text{ (J: psychological quantity, I: physical quantity, k: constant, and p: exponent)} \quad \cdots (1)$$

In Eq. (1), the psychological quantity J is a numerical value based on the response of the subject T1 when the subject T1 is stimulated. The physical quantity I is a physical quantity indicating the stimulus.

In the magnitude estimation method, because the physical quantity and the psychological quantity are indicated by Eq. (1), it is possible to infer the psychological quantity for the glare of the subject under unknown conditions using machine learning or the like. The magnitude estimation method is a method in which a visual reference target is defined and a sensory magnitude is directly estimated numerically through comparison with the visual reference target.

In the present embodiment, the psychological quantity J is a subjective evaluation value in an evaluation process performed on the basis of a response of the subject T1 about how many times or a fraction of the luminance of the visual target displayed after the blank image appears when the luminance of the reference glaring target displayed at the beginning of the examination is set to 100. The physical quantity I is the luminance ($cd/m^2$) when the luminance of the visual target is measured with a luminance meter.

In the measurement, as in the case of the double staircase method, the subject T1 sits in a chair so that the distance between the display device 3 and the eyes of the subject T1 is 1.0 m. A position of the fixed viewpoint presented in the fixed viewpoint image P51 is adjusted to match the eye height of the subject T1. A task of the subject T1 is to respond how many times or a fraction of the luminance of the second presented glaring target appears with respect to a reference glaring target presented at the beginning. The subject T1 responds, for example, by manipulating a mouse.

First, a reference glare illusion image P62 including a reference glaring target is presented for 1,000 msec after the fixed viewpoint image P61 is initially presented for 3,000 msec. Subsequently, a comparative glare illusion image P64 including a comparative visual target is presented for 300 msec after a blank image P63 is presented for 300 msec. After the comparative visual target is presented, a random dot screen P65 is presented as a mask stimulus for 30 sec to control an afterimage. While the random dot screen P65 is presented, the subject T1 responds to the luminance of the comparative visual target when the reference glaring target is 100.

As the used glaring target, 15 glare illusion images P2 including 15-step glaring targets shown in FIG. 10 were used. The glaring target G1 included in the glare illusion image P2 having the visual target number "12a" was used as the visual reference target. In the comparative visual target, 15-step glaring targets are presented in a random order. By randomizing the presentation order, the next comparative visual target to be presented to the subject T1 is not predicted.

In order to further improve the accuracy of the measurement, it is preferable to repeat the examination using the 15-step glaring target as one block a plurality of times. In that case, the psychological quantity J is an average value of the psychological quantity J for each physical quantity. The psychological quantity J is an example of the glare index A3.

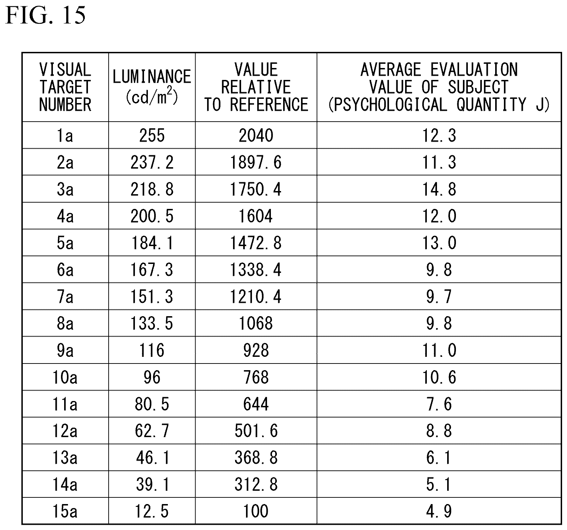

FIG. 15 is a diagram showing an example of an examination result in the magnitude estimation method according to the present embodiment. The luminance indicates the luminance of the center region of the glaring target of each visual target number. The visual reference target is a glaring target G1 having the visual target number "15a." The relative value for each visual target number is a relative value of the luminance of the center region of the glaring target of each target number when the luminance of the center region of the glaring target G1 of the visual target number "15a" is set to 100. An average evaluation value of the subject T1 is an average value for the number of trials of the brightness responded by the subject T1 for each glaring target with four trials.

In the visual function examination device 2, the constants k and the exponent p in the above-described Eq. (1) are calculated on the basis of the examination result in the magnitude estimation method as shown in FIG. 15 and a prescribed analysis program. The calculation process is mainly performed by the glare index calculation unit 203. If the constant k and the exponent p are calculated, a relationship between the psychological quantity and the physical quantity of the subject T1 can be represented as in Eq. (1).

Here, an example of an algorithm in which the glare index calculation unit 203 calculates the constant k and the exponent p will be described.

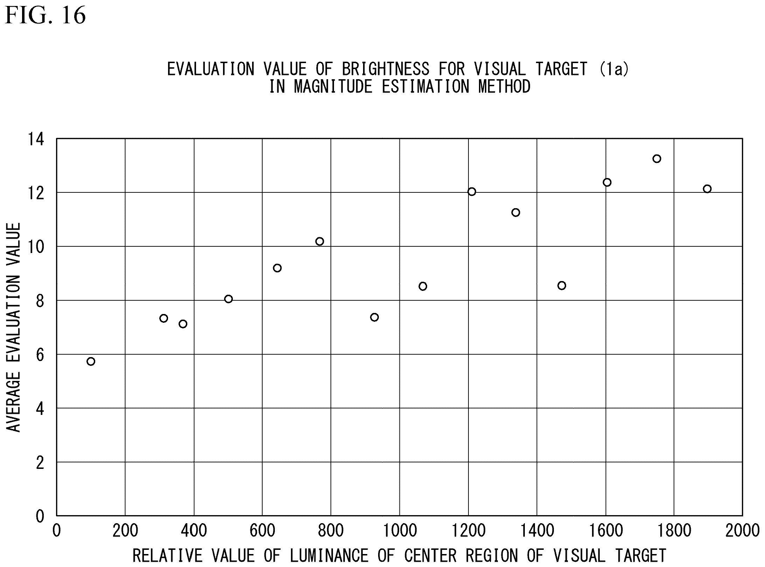

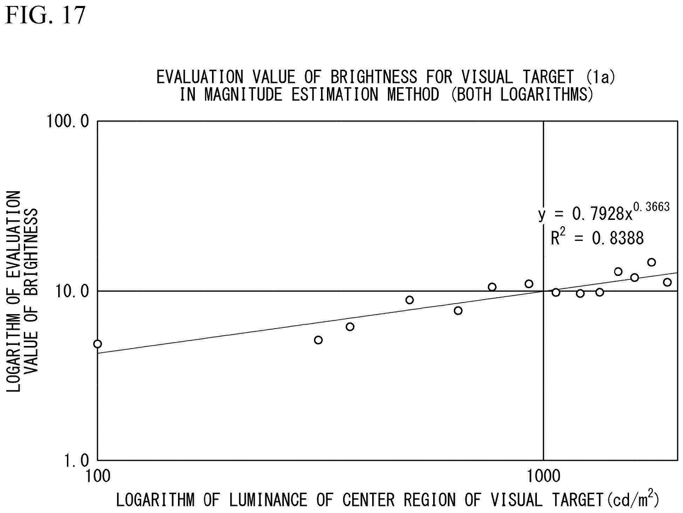

FIG. 16 shows a graph in which an average evaluation value is plotted with respect to the relative value of the luminance of the center region of the glaring target on the basis of the examination result in the magnitude estimation method shown in FIG. 15. FIG. 17 shows a graph of a case where graphs are plotted in both logarithms. When a power proximal curve is added to each point of the graph shown in FIG. 17 and a function indicating the curve is obtained in regression analysis, it becomes a function indicated according to Eq. (2).

[Math. 2]

$$J \; = \; 0.7928 \, I^{0.3663} \qquad \cdots (2)$$

The psychological quantity J corresponds to the average evaluation value and the physical quantity I corresponds to the relative value of the luminance of the center region of the glaring target. Also, it can be seen that the coefficient of determination of regression analysis is 0.94 and there is a strong correlation between the relative value of luminance and the average evaluation value as shown in FIG. 17. On the basis of Eq. (2), it is possible to estimate the psychological quantity for the glaring target that was not used in the actual measurement.

[Index calculation process of visual function examination device]

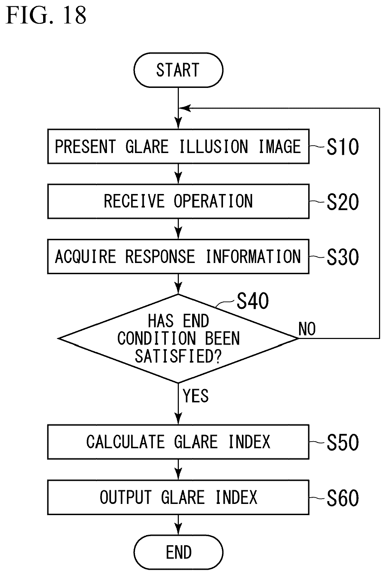

Next, an index calculation process in which the visual function examination device 2 calculates the glare index A3 will be described with reference to FIG. 18. FIG. 18 is a diagram showing an example of an index calculation process according to the present embodiment.

Step S10: The glare illusion image presentation unit 200 presents the glare illusion image A1. Here, the glare illusion image presentation unit 200 presents the glare illusion image A1 to the subject T1 by displaying the glare illusion image A1 on the display device 3. Here, in accordance with the examination method, the glare illusion image presentation unit 200 causes the glare illusion image A1 to be displayed on the display device 3 for a prescribed period of time.

Also, the glare illusion image presentation unit 200 causes the display device 3 to display a blank image as shown in FIG. 8 for a prescribed period of time in periods before and after the glare illusion image A1 is presented in accordance with the examination method. Also, the glare illusion image presentation unit 200 presents a fixed viewpoint image as shown in FIG. 8 before first presenting the glare illusion image A1.

Step S20: The operation reception unit 201 receives an operation in which the subject T1 inputs a response from the input device 4. The response is a response about glare felt by the subject T1 with respect to the glare illusion image A1 presented by the glare illusion image presentation unit 200. The response is a response according to the examination method. For example, when the examination method is a double staircase method, the response is a response as to whether or not the subject T1 has felt glare with respect to the glare illusion image A1 displayed on the display device 3.

Also, in step S10, after the entire plurality of glaring targets G1 are presented, the subject T1 may respond with a number of the glare illusion image A1 including the glaring target G1 perceived to be glare from among the presented glaring targets G1.

The operation reception unit 201 supplies the received response to the response information acquisition unit 202 as the response information A2.

Step S30: The response information acquisition unit 202 acquires the response of the subject T1 input by the operation received by the operation reception unit 201 as the response information A2. The response information acquisition unit 202 supplies the acquired response information A2 to the glare index calculation unit 203.

Here, the response of the subject T1 input by the operation received by the operation reception unit 201 is a plurality of responses to glare felt by the subject T1 with respect to a plurality of glaring targets G1 when the plurality of glaring targets G1 that cause the glare illusion to different degrees are presented in a prescribed order. Therefore, the response information acquisition unit 202 acquires the response information A2 indicating the plurality of responses. Here, the prescribed order in which the plurality of glaring targets G1 are presented may be the order of luminance of the visual target as in the case of the double staircase method or may be random as in the case of the magnitude estimation method.

Step S40: The glare illusion image presentation unit 200 determines whether or not an end condition is satisfied. Here, the end condition is a condition for ending the presentation of the glare illusion image A1. The end condition is a condition according to the examination method. For example, when the examination method is a double staircase method, the end condition is that the number of trials is a prescribed number (for example, 7) after the response indicating that glare was felt and the response indicating that glare was not felt are repeated.

When it is determined that the end condition has been satisfied (step S40; YES), the glare illusion image presentation unit 200 outputs a signal indicating that the end condition has been satisfied to the glare index calculation unit 203. The glare index calculation unit 203 executes the processing of step 50. On the other hand, when it is determined that the end condition has not been satisfied (step S40; NO), the glare illusion image presentation unit 200 executes the processing of step 10 again.

Step S50: The glare index calculation unit 203 calculates the glare index A3 on the basis of the response information A2 acquired by the response information acquisition unit 202. The glare index calculation unit 203 calculates the glare index A3 in accordance with the examination method. For example, when the examination method is a double staircase method, the glare index calculation unit 203 calculates an absolute threshold as the glare index A3 by calculating an average value between the luminance values of the center region of each of the ascending series and the descending series in the range in which the responses are repeated. The glare index calculation unit 203 may calculate a physical quantity from a measured psychological quantity on the basis of the above-described Eq. (1) and designate the physical quantity as the glare index A3.

The glare index calculation unit 203 supplies the calculated glare index A3 to the presentation unit 204.

Step S60: The presentation unit 204 outputs the glare index A3 calculated by the glare index calculation unit 203. The presentation unit 204 outputs, for example, the glare index A3 to the display device 3. The display device 3 displays the glare index A3 output from the presentation unit 204. The subject T1, an optician clerk, or an ophthalmologist can select lenses according to sensitivity of the subject T1 to glare as spectacle lenses provided to the subject T1 on the basis of the glare index A3 displayed on the display device 3. Also, the presentation unit 204 may output the glare index A3 to an external database.

As described above, the visual function examination device 2 ends the index calculation process.

As described above, the visual function examination device 2 according to the present embodiment includes a response information acquisition unit 202 and a glare index calculation unit 203.

The response information acquisition unit 202 acquires the response information A2 indicating a response about glare felt by the subject T1 to the visual target based on the glare illusion (the glaring target G1 in the present embodiment).

The glare index calculation unit 203 calculates an index for glare felt by the subject T1 (the glare index A3 in the present embodiment) on the basis of the response information A2 acquired by the response information acquisition unit 202.

According to this configuration, the visual function examination device 2 according to the present embodiment can calculate an index for the glare felt by the subject T1 on the basis of the response about the glare felt by the subject T1 to the visual target based on the glare illusion, such that the sensitivity to the glare of the subject T1 can be examined.

Here, in the visual function examination device 2 according to the present embodiment, because a visual target based on the glare illusion is used to examine the sensitivity of the subject T1 to glare, it is not necessary to actually use an index having prescribed luminance or higher. In this regard, when an index having prescribed luminance or higher for implementation is displayed on a small screen of a notebook PC, a smartphone, a tablet terminal, or the like, sufficient luminance may not be implemented. On the other hand, in the visual function examination device 2 according to the present embodiment, even if the screen on which the glaring target G1 is displayed is small, the subject T1 can feel glare greater than or equal to the actual glare on the basis of the glare illusion.

Also, the visual function examination device 2 according to the present embodiment further includes a glare illusion image presentation unit 200 and a response input operation reception unit (the operation reception unit 201 in the present embodiment).

The glare illusion image presentation unit 200 presents the glare illusion image A1 that is an image including a visual target (the glaring target G1 in the present embodiment).

The response input operation reception unit (the operation reception unit 201 in the present embodiment) receives an operation of inputting a response about the glare felt by the subject T1 to the glare illusion image A1 presented by the glare illusion image presentation unit 200.

Here, the response information acquisition unit 202 acquires a response about the glare felt by the subject T1 input by the operation received by the response input operation reception unit (the operation reception unit 201 in the present embodiment) as the response information A2.

According to this configuration, because the visual function examination device 2 according to the present embodiment can present the glaring target G1 as an image and can receive an operation of inputting a response about the glare felt by the subject T1, it is possible to perform a smooth examination process.

Also, in the visual function examination device 2 according to the present embodiment, the response information acquisition unit 202 acquires the response information A2 indicative of a plurality of responses about glare felt by the subject T1 with respect to a plurality of visual targets (glaring targets G1 in the present embodiment) when the plurality of visual targets (the glaring targets G1 in the present embodiment) that cause the glare illusion to different degrees are presented in a prescribed order. Also, the glare index calculation unit 203 calculates an index (the glare index A3 in the present embodiment) on the basis of the response information A2 acquired by the response information acquisition unit 202.

According to this configuration, because the visual function examination device 2 according to the present embodiment can calculate an index for the glare felt by the subject T1 on the basis of a plurality of responses about glare felt by the subject T1 with respect to the plurality of visual targets that cause the glare illusion to different degrees, the accuracy of the calculated index can be enhanced as compared with the case where a plurality of visual targets are not used.

Although an example in which a plurality of visual targets that cause the glare illusion to different degrees are presented has been described in the present embodiment, the present invention is not limited thereto. A single glaring target G1 may be presented of a single type of glaring target G1 may be presented a plurality of times.

(Modified examples of first embodiment)

Although an example of a case where the visual function examination device 2 and the display device 3 are provided in an optician shop, an ophthalmic clinic, or the like and the visual function of the subject T1 is examined in the visual function examination system 1 has been described in the present embodiment, the present invention is not limited thereto. The glare illusion image A1 is presented by a terminal device in an optician shop, an ophthalmic clinic, or the like and a recording device or a central processing unit (CPU) on the cloud may be used in each of a process of storing the glare illusion image A1 and a process of calculating the glare index A3.

The visual function examination system according to the present modified example is referred to as a visual function examination system 1a. Also, components identical to those of the first embodiment described above may be denoted by the same reference signs and descriptions of the same components and operations may be omitted.

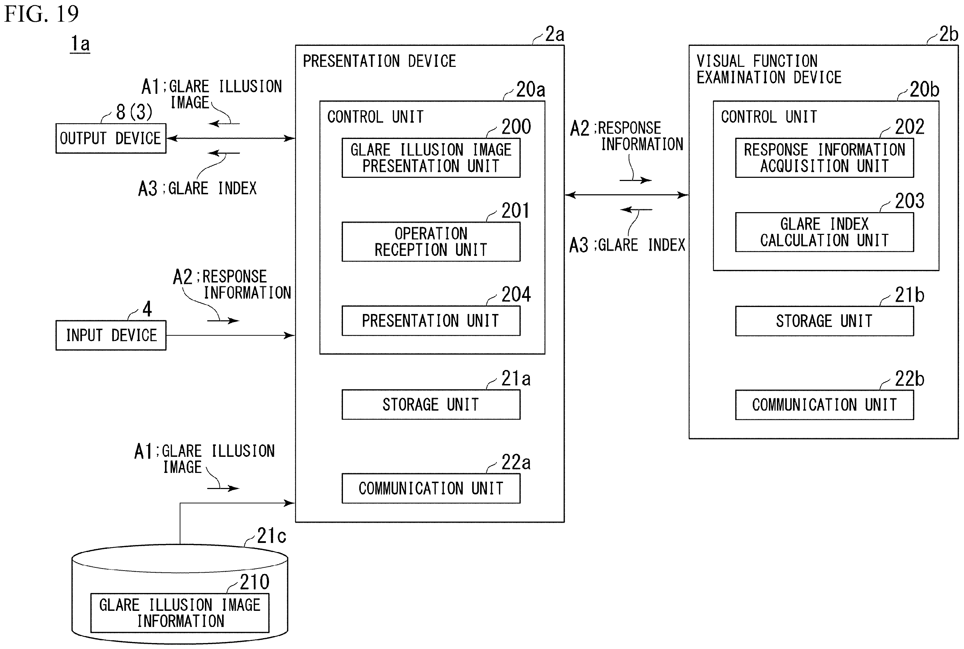

FIG. 19 is a diagram showing an example of a mechanical configuration of the visual function examination system 1a according to the present modified example. The visual function examination system 1a according to the present modified example includes a presentation device 2a, a visual function examination device 2b, a display device 3, and an input device 4. In the visual function examination system 1a, a functional unit provided in the visual function examination device 2 described above is provided separately into the presentation device 2a and the visual function examination device 2b.

The presentation device 2a is a PC as an example. As an example, the presentation device 2a is provided in an optician shop, an ophthalmic clinic, or the like. The presentation device 2a may be a smartphone, a tablet terminal, or the like. When the presentation device 2a is a smartphone or a tablet terminal, the display device 3 and the input device 4 are provided to be integrated with the presentation device 2a as a touch panel. Also, the presentation device 2a may be a head-mounted display.

The presentation device 2a includes a control unit 20a, a storage unit 21a, and a communication unit 22a.

The control unit 20a includes a glare illusion image presentation unit 200, an operation reception unit 201, and a presentation unit 204. Here, the glare illusion image presentation unit 200 acquires the glare illusion image A1 from a database 21c provided on the cloud and causes the display device 3 to display the glare illusion image A1 that has been acquired. The presentation unit 204 outputs the glare index A3 calculated by the visual function examination device 2b to the display device 3.

The storage unit 21a stores various types of information. The glare illusion image A1 may not be stored in the storage unit 21a.

The communication unit 22a transmits and receives various types of information via a wireless network. The communication unit 22a includes a communication interface (I/F) for performing communication via the wireless network. The presentation device 2a communicates with the visual function examination device 2b and the database 21c via the communication unit 22a.

The visual function examination device 2b includes a control unit 20b, a storage unit 21b, and a communication unit 22b.

The control unit 20b includes a response information acquisition unit 202 and a glare index calculation unit 203. The response information acquisition unit 202 acquires the response information A2 from the operation reception unit 201 provided in the presentation device 2a. The glare index calculation unit 203 outputs the calculated glare index A3 to the presentation unit 204 provided in the presentation device 2a.

The storage unit 21b stores various types of information.

The communication unit 22b transmits and receives various types of information via the wireless network. The communication unit 22a includes a communication interface (I/F) for performing communication via the wireless network. The visual function examination device 2b communicates with the presentation device 2a via the communication unit 22a.

The database 21c is a database for storing the glare illusion image information 210. The database 21c supplies the glare illusion image A1 to the presentation device 2a in response to a request from the presentation device 2a.

(Second embodiment)

Hereinafter, a second embodiment of the present invention will be described in detail with reference to the drawings.

In the above-described first embodiment, a case where the glare illusion image A1 is generated in advance and stored in the storage unit in the visual function examination system 1 has been described. In the present embodiment, a case where the visual function examination system generates a glare illusion image A1 on the basis of input parameters will be described. Also, components identical to those of the first embodiment described above may be denoted by the same reference signs and descriptions of the same components and operations may be omitted.

The visual function examination system according to the present embodiment is referred to as a visual function examination system 1d and the visual function examination device is referred to as a visual function examination device 2d.

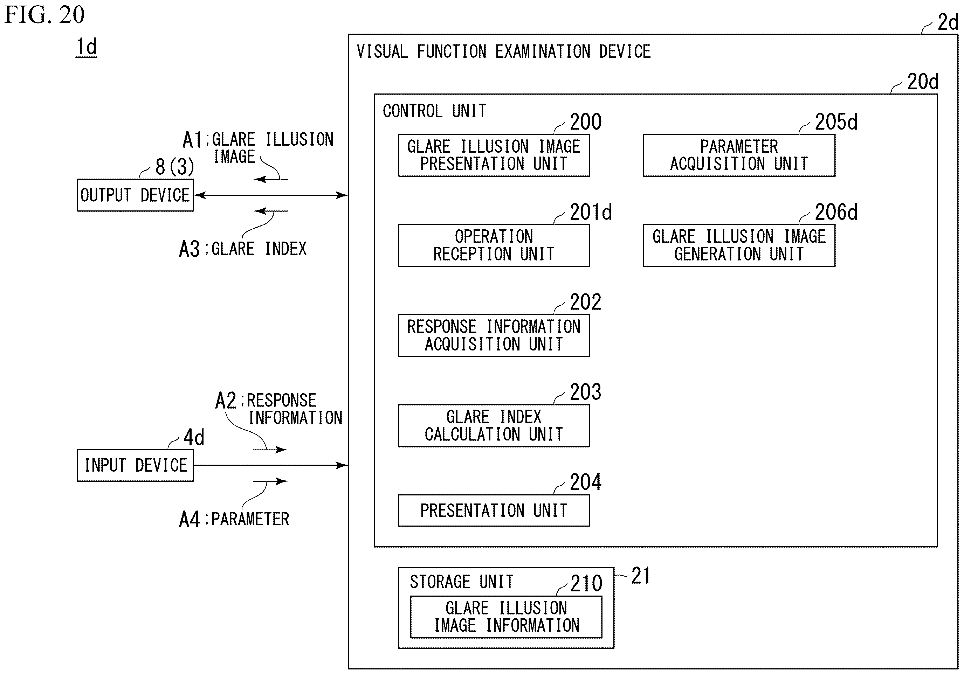

FIG. 20 is a diagram showing an example of a functional configuration of a visual function examination system 1d according to the present embodiment. The visual function examination device 2d includes a control unit 20d and a storage unit 21.

The control unit 20d includes a glare illusion image presentation unit 200, an operation reception unit 201d, a response information acquisition unit 202, a glare index calculation unit 203, a presentation unit 204, a parameter acquisition unit 205d, and a glare illusion image generation unit 206d.

The operation reception unit 201d receives various types of operations from an input device 4d. The various types of operations received by the operation reception unit 201d include an operation of inputting parameters for creating a glare illusion image A1.

The parameter acquisition unit 205d acquires a parameter A4 input to the input device 4d from the operation reception unit 201. The parameter A4 is a parameter for generating the glare illusion image A1. The parameter A4 includes a center region parameter A41 and an induction region parameter A42. The center region parameter A41 is a parameter for the center region included in the glare illusion image A1. The induction region parameter A42 is a parameter for the induction region included in the glare illusion image A1. A specific example of the parameter A4 is a parameter shown in FIG. 6.

A glare illusion image generation unit 206d generates the glare illusion image A1 on the basis of the parameter A4 acquired by the parameter acquisition unit 205d.

Although an example in which the center region R21 and the induction region R22 are arranged concentrically in the glare illusion image A1 according to the present embodiment has described, the present invention is not limited thereto. For example, the shape of the center region may be a polygon or an ellipse. Although the induction region is arranged to surround the periphery of the center region in the glare illusion image A1, the induction region may surround a part of the outer periphery of the center region without having to surround all of the outer periphery of the center region.

Although an example in which the center region and the induction region are grayscale images in the glare illusion image A1 according to the present embodiment has been described, the present invention is not limited thereto. The center region and the induction region may have a color other than black and white.

In relation to the luminance gradient of the induction region, it is necessary to consider the gamma adjustment value of the display of the display device 3. By decreasing the gamma value, the average luminance of the glaring target G1 can be lowered. By increasing the gamma value, the average luminance of the glaring target G1 can be raised.

The luminance gradient of the induction region may be either a gradient in which the luminance increases or a gradient in which the luminance decreases toward the center region. Also, the gradient in which the luminance increases has a larger glare illusion effect than the gradient in which the luminance decreases.

A background may be other than a background of the glare illusion image A1 according to the present embodiment. The background may be any background, but it is preferable that the luminance of the background be small to reduce the average luminance of the entire screen.

As described above, the visual function examination device 2d according to the present embodiment includes a parameter acquisition unit 205d and a glare illusion image generation unit 206d.

The parameter acquisition unit 205d acquires a center region parameter A41, which is a parameter for generating the glare illusion image A1 and a parameter for the center region R21 included in the glare illusion image A1, and an induction region parameter A42, which is a parameter for the induction region R22 included in the glare illusion image A1.

The glare illusion image generation unit 206d generates the glare illusion image A1 on the basis of the center region parameter A41 and the induction region parameter A42 acquired by the parameter acquisition unit 205d.

According to this configuration, because the visual function examination device 2d according to the present embodiment can generate the glare illusion image A1, the trouble and cost of providing the glare illusion image A1 in advance can be saved.

(Third embodiment)

Hereinafter, a third embodiment of the present invention will be described in detail with reference to the drawings.

In the above-described first embodiment, a case where the visual function examination system calculates the glare index A3 has been described. In the present embodiment, a case where a visual function examination system determines spectacle lenses suitable for the subject T1 on the basis of the calculated glare index A3 will be described. Also, components identical to those of the first embodiment described above may be denoted by the same reference signs and descriptions of the same components and operations may be omitted.

The visual function examination system 1 according to the present embodiment is referred to as a visual function examination system 1e.

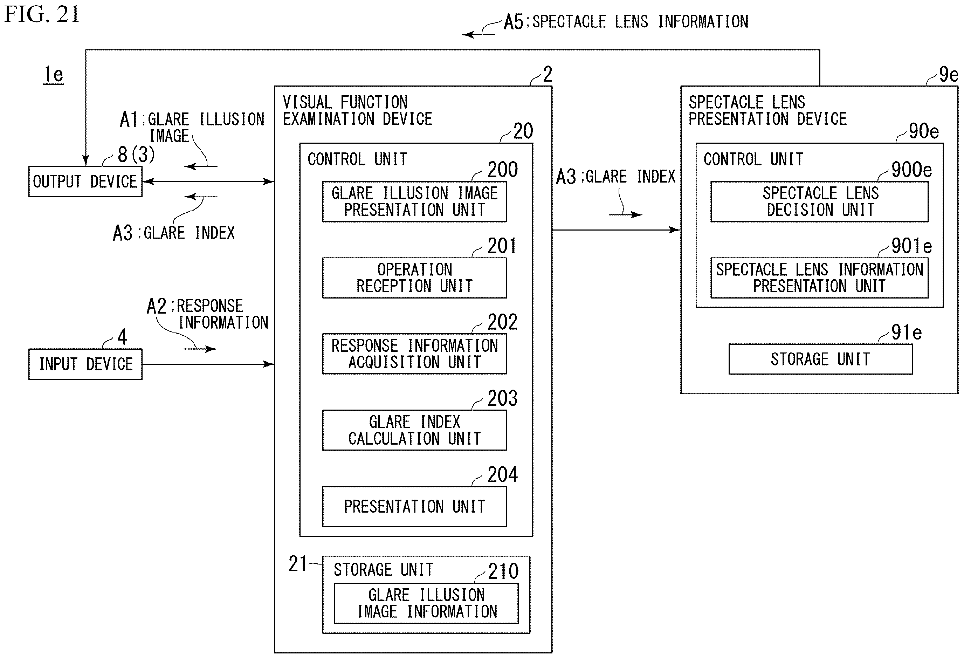

FIG. 21 is a diagram showing an example of a functional configuration of the visual function examination system 1e according to the present embodiment. The visual function examination system 1e includes a visual function examination device 2, a display device 3, an input device 4, and a spectacle lens presentation device 9e. The spectacle lens presentation device 9e may be provided to be integrated with the visual function examination device 2. That is, each functional unit provided in the spectacle lens presentation device 9e may be provided in the visual function examination device 2.

The spectacle lens presentation device 9e is a PC as an example. The spectacle lens presentation device 9e is provided in an optician shop, an ophthalmic clinic, or the like together with the visual function examination device 2. The spectacle lens presentation device 9e includes a control unit 90e and a storage unit 91e.

The control unit 90e includes a spectacle lens decision unit 900e and a spectacle lens information presentation unit 901e.

The spectacle lens decision unit 900e decides on spectacle lenses L suitable for the subject T1 on the basis of the glare index A3 calculated by the visual function examination device 2. Here, the spectacle lens decision unit 900e may decide on a parameter for the spectacle lenses L1 as a value suitable for the subject T1 or select spectacle lenses L suitable for the subject T1 from a plurality of spectacle lenses L provided in advance.

The spectacle lens information presentation unit 901e presents information about the spectacle lenses L decided on by the spectacle lens decision unit 900e. Information about the spectacle lenses L decided on by the spectacle lens decision unit 900e is referred to as spectacle lens information A5. The spectacle lens information presentation unit 901e outputs, for example, the spectacle lens information A5 to the display device 3. The display device 3 displays information about the spectacle lenses L indicated in the spectacle lens information A5 output from the spectacle lens information presentation unit 901e.

For example, the spectacle lens decision unit 900e decides on the spectacle lenses L suitable for the subject T1 as follows. On the basis of Eq. (1) indicating the relationship between physical and psychological quantities described above and a relational expression between the background luminance and the light source luminance indicated by Expression (3), it is possible to estimate sensitivity to glare (glare sensation) when the subject T1 observes a light source having certain luminance in a certain environment.

[Math. 3]

$$Ls \propto k*Lb^p \text{ (Ls: luminance of light source, Lb: background luminance, k:}$$

$$\text{constant, and p: exponent)} \qquad \cdots (3)$$

In a case where the spectacle lenses suitable for eye characteristics of the subject T1 are produced, information about the lifestyle of the subject T1 is further obtained to know in what environment the subject T1 wears spectacle lenses. By estimating the sensitivity (glare sensation) to glare when the subject T1 observes a light source having certain luminance in a certain environment, it is possible to produce the spectacle lenses suitable for the subject T1.

For example, when it is determined that the subject T1 is prone to feeling glare as a result of the above-described examination and spectacle lenses suitable for outdoor work are produced, lenses and spectacle lens frames necessary for controlling light entering the eyes can be considered.

As another example, when the result of the above-described examination indicates that it is determined that the subject T1 is prone to glare, spectacle lenses to provide better vision are required to drive a car at night. In such a case, it is considered necessary to have spectacle lenses that can secure a field of view at night and avoid glare on the headlamps of oncoming vehicles. As such spectacle lenses, it is conceivable to propose lenses containing a colorant that can absorb a specific wavelength more.

For example, environmental background luminance information, which is a table in which an environment and background luminance information in the environment are associated, is stored in the storage unit 91e in advance. The spectacle lens decision unit 900e determines in what environment the subject T1 wears spectacle lenses from information about the lifestyle of the subject T1. The spectacle lens decision unit 900e estimates the sensitivity to the glare (glare sensation) in the case where the subject T1 has observed a light source having certain luminance in the determined environment on the basis of the glare index A3 calculated by the visual function examination device 2, the determined environment, and the environment background luminance information. The spectacle lens decision unit 900e decides on the spectacle lenses L suitable for the subject T1 on the basis of the estimated sensitivity (glare sensation) to glare.

As described above, the visual function examination system 1e according to the present embodiment includes the visual function examination device 2 and the spectacle lens presentation device 9e.

The spectacle lens presentation device 9e includes the spectacle lens decision unit 900e and the spectacle lens information presentation unit 901e.

The spectacle lens decision unit 900e decides on the spectacle lenses L suitable for the subject T1 on the basis of the index calculated by the visual function examination device 2 (the glare index A3 in the present embodiment).

The spectacle lens information presentation unit 901e presents information about spectacle lens L decided on by the spectacle lens decision unit 900e (the spectacle lens information A5 in the present embodiment).

According to this configuration, in the visual function examination system 1e according to the present embodiment, the spectacle lenses L suitable for the subject T1 can be decided on based on the sensitivity of the subject T1 to glare, such that the spectacle lenses L suitable for the subject T1 can be presented on the basis of the sensitivity.

(Fourth embodiment)

Hereinafter, a fourth embodiment of the present invention will be described in detail with reference to the drawings.

In the third embodiment, a case in which the visual function examination system decides on spectacle lenses suitable for the subject T1 on the basis of the calculated glare index A3 has been described. In the present embodiment, a case where a visual function examination system simulates the appearance of a glaring target G1 when the subject T1 wears spectacle lenses will be described. Also, components identical to those of the first embodiment described above may be denoted by the same reference signs and descriptions of the same components and operations may be omitted.

The visual function examination system according to the present embodiment is referred to as a visual function examination system 1f.

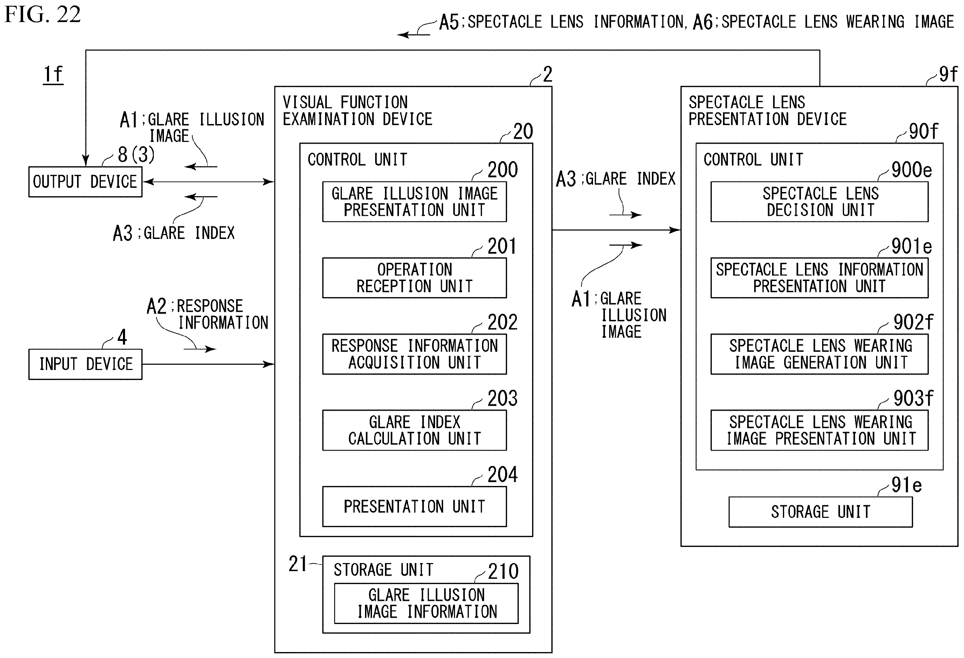

FIG. 22 is a diagram showing an example of a functional configuration of the visual function examination system 1f according to the present embodiment. The visual function examination system 1f includes a visual function examination device 2, a display device 3, an input device 4, and a spectacle lens presentation device 9f. Also, the spectacle lens presentation device 9f may be provided to be integrated with the visual function examination device 2. That is, each functional unit provided in the spectacle lens presentation device 9f may be provided in the visual function examination device 2.

The spectacle lens presentation device 9f includes a control unit 90f and a storage unit 91e. The control unit 90f includes a spectacle lens decision unit 900e, a spectacle lens information presentation unit 901e, a spectacle lens wearing image generation unit 902f, and a spectacle lens wearing image presentation unit 903f. Also, the spectacle lens wearing image generation unit 902f and the spectacle lens wearing image presentation unit 903f may be provided separately from the spectacle lens presentation device 9f in the visual function examination system 1f. For example, the spectacle lens wearing image generation unit 902f and the spectacle lens wearing image presentation unit 903f may be provided in the visual function examination device 2 or may be provided in a terminal device other than the visual function examination device 2 or the spectacle lens presentation device 9f.

The spectacle lens wearing image generation unit 902f generates a spectacle lens wearing image A6 on the basis of spectacle lens information A5 about the spectacle lenses L decided on by the spectacle lens decision unit 900e. The spectacle lens wearing image A6 is a glare illusion image including the glaring target G1 that appears when the subject T1 wears the spectacle lenses L.

The spectacle lens wearing image presentation unit 903f presents the spectacle lens wearing image A6 generated by the spectacle lens wearing image generation unit 902f. The spectacle lens wearing image presentation unit 903f presents the spectacle lens wearing image A6 by, for example, causing the display device 3 to display the spectacle lens wearing image A6. As an example, the spectacle lens wearing image presentation unit 903f causes the display device 3 to display the spectacle lens wearing image A6 and the glare illusion image A1 side by side.

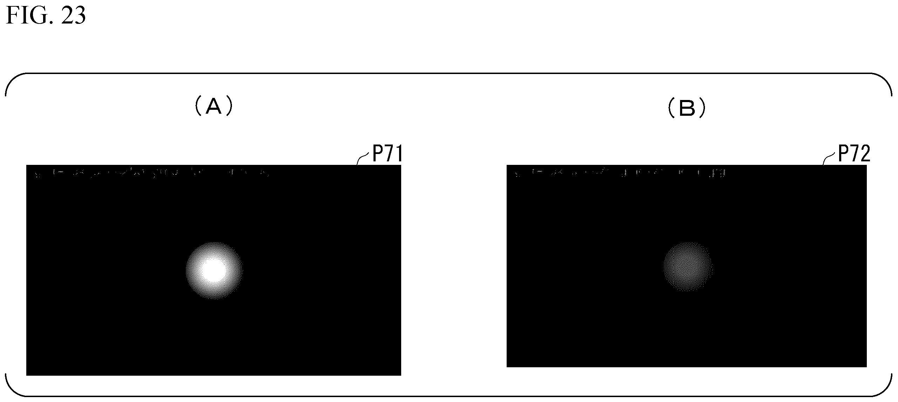

Here, the spectacle lens wearing image A6 presented by the spectacle lens wearing image presentation unit 903f will be described with reference to FIG. 23. FIG. 23 is a diagram showing an example of the spectacle lens wearing image A6 according to the present embodiment. In FIG. 23(A), a glare illusion image P71 is shown as an example of a glare illusion image A1 displayed side by side with the spectacle lens wearing image A6. In FIG. 23(B), a spectacle lens wearing image P72 is shown as an example of the spectacle lens wearing image A6.

The spectacle lens wearing image presentation unit 903f selects the glare illusion image A1 displayed side by side with the spectacle lens wearing image A6 on the basis of the glare index A3 calculated by the glare index calculation unit 203. For example, the spectacle lens wearing image presentation unit 903f selects a glare illusion image A1 having a center region of luminance close to an absolute threshold value calculated in the above-described double staircase method from among glare illusion images A1 included in the glare illusion image information 210 stored in the storage unit 21 of the visual function examination device 2. The spectacle lens wearing image presentation unit 903f acquires the selected glare illusion image A1 from the visual function examination device 2.

In the present embodiment, the spectacle lens wearing image A6 includes a transmittance cut ratio as a parameter. The spectacle lens wearing image generation unit 902f generates a spectacle lens wearing image A6 on the basis of the appearance of the glaring target G1 when a transmittance cut identical to that of the lens color density determined on the basis of the lifestyle and life habit of the subject T1 is applied. Here, the lens color density determined on the basis of the lifestyle and life habit of the subject T1 is decided on based on the glare index A3 calculated by the visual function examination device 2, the determined environment, and the environmental background luminance information as in the determination of the spectacle lenses L suitable for the subject T1 in the third embodiment.

The spectacle lens wearing image presentation unit 903f sequentially presents a plurality of spectacle lens wearing images A6 while changing the transmittance cut ratio. In a process in which the spectacle lens wearing image presentation unit 903f sequentially presents the plurality of spectacle lens wearing images A6, the luminance around the display device 3 and the subject T1 is preferably constant.

The subject T1 is able to confirm how much the spectacle lenses to be purchased can reduce glare by comparing the glare illusion image P71 and the spectacle lens wearing image P72 displayed on the display device 3.

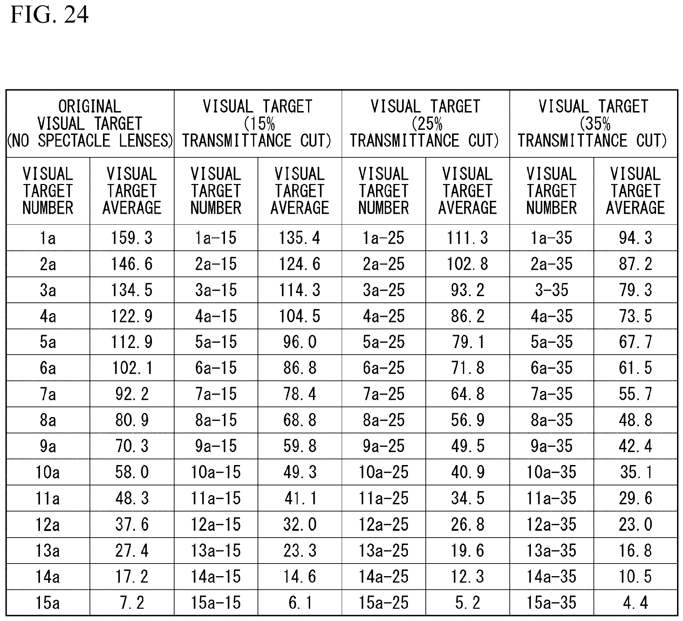

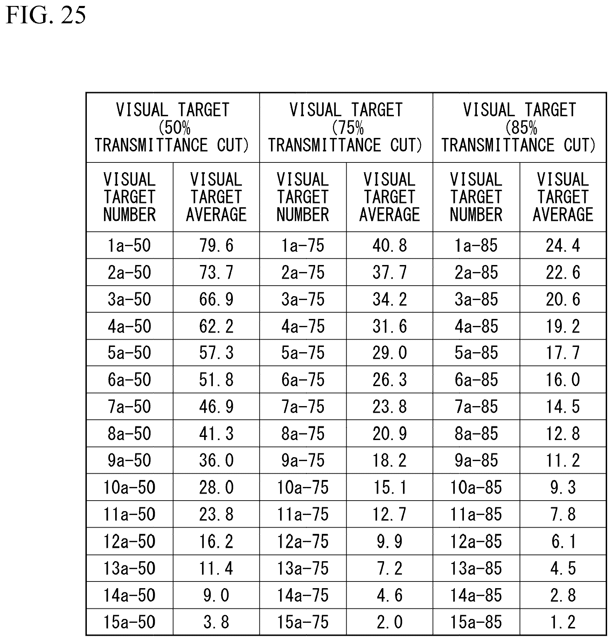

In FIGS. 24 and 25, the average luminance of the glaring target G1 included in the spectacle lens wearing image A6 when the transmittance cut ratio has been variously changed is shown. In FIGS. 24 and 25, the average luminance of the glaring target G1 included in the spectacle lens wearing image A6 for which a transmittance cut-specific process is performed is shown with respect to the glare illusion images A1 having the visual target numbers "1a" to "15a" shown in FIG. 10. Here, there are five transmission cut ratios: 15, 25, 35, 75, and 85%. Also, the average luminance of the glaring target G1 is the average luminance of the center region and the induction region.

For example, the spectacle lens wearing image A6 close to the absolute threshold value measured in the double staircase method is designated as the spectacle lens wearing image A6 having the visual target number "6a." In this case, the subject T1 can confirm the glare and visibility of the visual target by staring at the spectacle lens wearing images A6 of the visual target numbers "6a-15," "6a-25," "6a-35," "6a-50," "6a-75," and "6a-85."

As described above, in the visual function examination system 1f according to the present embodiment, the spectacle lens presentation device 9f includes a spectacle lens wearing image generation unit 902f and a spectacle lens wearing image presentation unit 903f.

The spectacle lens wearing image generation unit 902f generates a spectacle lens wearing image A6 that is a glare illusion image including a visual target (a glaring target G1 in the present embodiment) that can be seen when the spectacle lenses L are worn by the subject T1 on the basis of information about the spectacle lenses L determined by the spectacle lens decision unit 900e (spectacle lens information A5 in the present embodiment).

The spectacle lens wearing image presentation unit 903f presents the spectacle lens wearing image A6 generated by the spectacle lens wearing image generation unit 902f.

According to this configuration, in the visual function examination system If according to the present embodiment, the subject T1 can simulate an extent to which glare can be reduced in a case where the spectacle lenses L are worn by the subject T1 when the subject T1 views the spectacle lens wearing image A6. In the visual function examination system 1f, it is possible to decide on a lens color and a transmittance cut necessary for the lifestyle and living environment of the subject T1 through a simulation of the appearance when the spectacle lenses L are worn. The subject T1 can verify specifications of the spectacle lenses to be purchased through the simulation.

(Fifth embodiment)

Hereinafter, a fifth embodiment of the present invention will be described in detail with reference to the drawings.

Although an example in which the visual function examination device includes a glare illusion image presentation unit and the glaring target G1 is included in the glare illusion image A1 has been described in each embodiment described above, the present invention is not limited thereto. In the present embodiment, a case where the glaring target G1 is presented as a printed matter on which the glaring target G1 is printed will be described. Also, components identical to those of the first embodiment described above may be denoted by the same reference signs and descriptions of the same components and operations may be omitted.

The visual function examination system according to the present embodiment is referred to as a visual function examination system 1g.

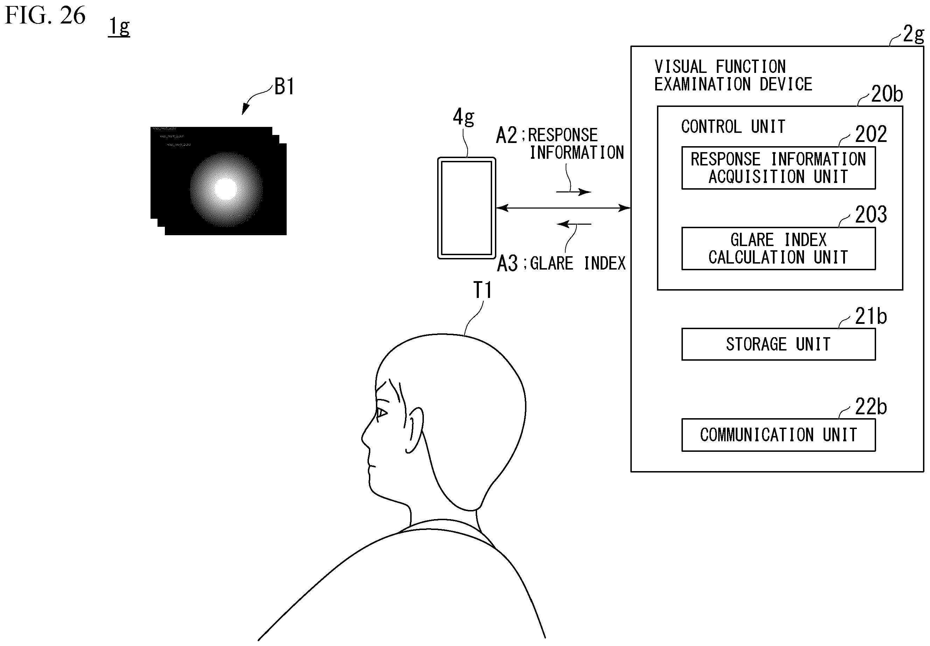

FIG. 26 is a diagram showing an example of a configuration of the visual function examination system 1g according to the present embodiment. The visual function examination system 1g includes a visual function examination device 2g and an input device 4g.

A booklet B1 includes a plurality of printed matters on which a plurality of glaring targets G1 that cause the glare illusion to different degrees are printed.

The booklet B1 is sent in advance for example, from an optician shop or an ophthalmic clinic to the subject T1. Also, a staff member may show the booklet B1 to the subject T1 in the optician shop or the ophthalmic clinic. The subject T1 stares at the glaring target G1 printed on each of the printed matters while turning over the plurality of printed matters included in the booklet B1. The subject T1 inputs a response about the glare felt by the subject T1 to the printed glaring target G1 from the input device 4g. For example, the subject T1 sequentially inputs a response about the glare felt for each of the plurality of glaring target G1 from the input device 4g. The response may indicate whether or not there is glare or may indicate a number of the glaring target G1 perceived to be glare.

The input device 4g is, for example, a smartphone or a tablet terminal. The input device 4 communicates with the visual function examination device 2g. For example, the function provided in the visual function examination device 2g is similar to the function provided in the visual function examination device 2b of FIG. 19 described above. The response information acquisition unit 202 acquires the response information A2 transmitted from the input device 4g. The visual function examination device 2g outputs the glare index A3 calculated by the glare index calculation unit 203 to the input device 4g.

As described above, a plurality of visual targets (the glaring targets G1 in the present embodiment), which are based on the glare illusion and causes the glare illusion to different degrees, are printed on a plurality of printed matters (the booklet B1 in the present embodiment) according to the present embodiment.

By using a plurality of printed matters according to the present embodiment (the booklet B1 in the present embodiment), even if the subject T1 is not good at operating the PC or the subject T1 is not in an environment where the PC can be used, the subject T1 can examine the sensitivity to the glare while turning over pages of the booklet B1.

Also, in some of the visual function examination devices 2, 2b, 2d, and 2g, the presentation device 2a, and the spectacle lens presentation devices 9e and 9f in the above-described embodiment, for example, the glare illusion image presentation unit 200, the operation reception unit 201, the response information acquisition unit 202, the glare index calculation unit 203, the presentation unit 204, the parameter acquisition unit 205d, the glare illusion image generation unit 206d, the spectacle lens decision unit 900e, the spectacle lens information presentation unit 901e, the spectacle lens wearing image generation unit 902f, and the spectacle lens wearing image presentation unit 903f may be implemented by a computer. In this case, the control functions may be implemented by recording a program for implementing the control functions on a computer-readable recording medium and causing a computer system to read and execute the program recorded on the recording medium. Also, it is assumed that the "computer system" used herein is a computer system built into the visual function examination devices 2, 2b, 2d, and 2g, the presentation device 2a, and the spectacle lens presentation devices 9e and 9f, and includes an operating system (OS) and hardware such as peripheral equipment. Also, the "computer-readable recording medium" refers to a flexible disk, a magnetooptical disc, a read only memory (ROM), a portable medium such as a compact disc (CD)-ROM, or a storage device such as a hard disk embedded in the computer system. Further, the "computer-readable recording medium" may include a computer-readable recording medium for dynamically holding the program for a short time period as in a communication line when the program is transmitted via a network such as the Internet or a communication circuit such as a telephone circuit and a computer-readable recording medium for holding the program for a given time period as in a volatile memory inside the computer system serving as a server or a client when the program is transmitted. Also, the above-described program may be a program for implementing some of the above-described functions. Further, the above-described program may be a program capable of implementing the above-described function in combination with a program already recorded on the computer system.

Also, some or all of the visual function examination devices 2, 2b, 2d, and 2g, the presentation device 2a, and the spectacle lens presentation devices 9e and 9f in the above-described embodiment may be implemented as an integrated circuit such as a large scale integration (LSI) circuit, the functional blocks of the visual function examination devices 2, 2b, 2d, and 2g, the presentation device 2a, and the spectacle lens presentation devices 9e and 9f may be implemented by individual processors or may be implemented by an integrated processor in which some or all thereof are integrated. Also, an integrated circuit implementation method may be implemented not only by an LSI circuit but also by a dedicated circuit or a general-purpose processor. Also, in the case where the integrated circuit technology which is substituted for an LSI circuit appears due to the advance of the semiconductor technology, an integrated circuit based on the technology may be used.

Although embodiments of the present invention have been described in detail above with reference to the drawings, specific configurations are not limited to the embodiments and various design changes and the like may also be made without departing from the scope of the present invention.

[Reference Signs List]

1, 1a, 1d, 1e, 1f Visual function examination system
2, 2b, 2d Visual function examination device
202 Response information acquisition unit
203 Glare index calculation unit
G1 Glaring target
A1 Glare illusion image
A2 Response information
A3 Glare index

T1 Subject

**Claims**

1. A visual function examination device comprising:

   a response information acquisition unit configured to acquire response information indicating a response about glare felt by a subject to a visual target based on a glare illusion; and
   a glare index calculation unit configured to calculate an index for the glare felt by the subject on the basis of the response information acquired by the response information acquisition unit.

2. The visual function examination device according to claim 1, further comprising:

   a glare illusion image presentation unit configured to present a glare illusion image that is an image including the visual target; and
   a response input operation reception unit configured to receive an operation of inputting the response to the glare illusion image presented by the glare illusion image presentation unit,
   wherein the response information acquisition unit acquires the response input by the operation received by the response input operation reception unit as the response information.

3. The visual function examination device according to claim 2, further comprising:

   a parameter acquisition unit configured to acquire a center region parameter that is a parameter for a center region included in the glare illusion image as a parameter for generating the glare illusion image, and an induction region parameter that is a parameter for an induction region included in the glare illusion image; and
   a glare illusion image generation unit configured to generate the glare illusion image on the basis of the center region parameter and the induction region parameter acquired by the parameter acquisition unit.

4. The visual function examination device according to claim 2 or 3,

   wherein the response information acquisition unit acquires the response information indicating a plurality of responses about the glare felt by the subject for a plurality of visual targets when the plurality of visual targets that cause the glare illusion to different degrees are presented in a prescribed order, and
   wherein the index calculation unit calculates the index on the basis of the response information acquired by the response information acquisition unit.

5. A spectacle lens presentation system comprising:

   a visual function examination device according to any one of claims 1 to 4; and
   a spectacle lens presentation device,
   wherein the spectacle lens presentation device includes
   a spectacle lens decision unit configured to decide on spectacle lenses suitable for the subject on the basis of the index calculated by the visual function examination device; and
   a spectacle lens information presentation unit configured to present information about the spectacle lenses decided on by the spectacle lens decision unit.

6. The spectacle lens presentation system according to claim 5, wherein the spectacle lens presentation device further includes

   a spectacle lens wearing image generation unit configured to generate a spectacle lens wearing image that is a glare illusion image including the visual target viewed when the subject has worn the spectacle lenses on the basis of the information about the spectacle lenses decided on by the spectacle lens decision unit; and
   a spectacle lens wearing image presentation unit configured to present the spectacle lens wearing image generated by the spectacle lens wearing image generation unit.

7. A plurality of printed matters on which a plurality of visual targets, which are based on a glare illusion and cause the glare illusion to different degrees, are printed.

8. A visual function examination method comprising:

   acquiring response information indicating a response about glare felt by a subject to a visual target based on a glare illusion; and
   calculating an index for the glare felt by the subject on the basis of the acquired response information.

9. A spectacle lens presentation method comprising:

   acquiring response information indicating a response about glare felt by a subject to a visual target based on a glare illusion;
   calculating an index for the glare felt by the subject on the basis of the acquired response information;
   deciding on spectacle lenses suitable for the subject on the basis of the calculated index; and
   presenting information about the decided spectacle lenses.

10. A program for causing a computer to execute:

    a response information acquisition step of acquiring response information indicating a response about glare felt by a subject to a visual target based on a glare illusion; and
    a glare index calculation step of calculating an index for the glare felt by the subject on the basis of the response information acquired in the response information acquisition step.

FIG. 1

FIG. 2

R11

R12

FIG. 3

1

INFORMATION FLOW ----→ CONTROL FLOW

FIG. 4

FIG. 5

FIG. 6

| CENTER REGION | INDUCTION REGION | BACKGROUND |
|---|---|---|
| RADIUS (UNITS OF PIXELS) CENTER COORDINATES(x、y) LUMINANCE RGB VALUE (HEXACODE) EXAMPLE:#FFFFFF | RADIUS (UNITS OF PIXELS) LARGEST LUMINANCE RGB VALUE SMALLEST LUMINANCE RGB VALUE GAUSSIAN FUNCTION, GAMMA CORRECTION VALUE | RESOLUTION OF SCREEN (x,y) LUMINANCE RGB VALUE |

FIG. 7

| CENTER REGION | INDUCTION REGION | BACKGROUND |
|---|---|---|
| RADIUS:53 PIXELS POSITION COORDINATES : (960,540) RGB VALUE :#FFFFFF~#4A4A4A | RADIUS:53 PIXELS LARGEST LUMINANCE :#FFFFFF SMALLEST LUMINANCE :#000000 GAMMA CORRECTION VALUE :2.0 | RESOLUTION OF SCREEN (1920,1080) RGB VALUE:#FFFFFF |

FIG. 8

## FIG. 9

DOUBLE STAIRCASE METHOD

FIG. 10

| VISUAL TARGET NUMBER | CENTER REGION | INDUCTION REGION | | VISUAL TARGET AVERAGE | BACKGROUND |
|---|---|---|---|---|---|
| | | MAXIMUM VALUE | MINIMUM VALUE | | |
| 1a | 255. 0 | 255. 0 | 0. 7 | 159. 3 | 0. 7 |
| 2a | 237. 2 | 237. 2 | 0. 7 | 146. 6 | 0. 7 |
| 3a | 218. 8 | 218. 8 | 0. 7 | 134. 5 | 0. 7 |
| 4a | 200. 5 | 200. 5 | 0. 7 | 122. 9 | 0. 7 |
| 5a | 184. 1 | 184. 1 | 0. 7 | 112. 9 | 0. 7 |
| 6a | 167. 3 | 167. 3 | 0. 7 | 102. 1 | 0. 7 |
| 7a | 151. 3 | 151. 3 | 0. 7 | 92. 2 | 0. 7 |
| 8a | 133. 5 | 133. 5 | 0. 7 | 80. 9 | 0. 7 |
| 9a | 116. 0 | 116. 0 | 0. 7 | 70. 3 | 0. 7 |
| 10a | 96. 0 | 96. 0 | 0. 7 | 58. 0 | 0. 7 |
| 11a | 80. 5 | 80. 5 | 0. 7 | 48. 3 | 0. 7 |
| 12a | 62. 7 | 62. 7 | 0. 7 | 37. 6 | 0. 7 |
| 13a | 46. 1 | 46. 1 | 0. 7 | 27. 4 | 0. 7 |
| 14a | 39. 1 | 39. 1 | 0. 7 | 17. 2 | 0. 7 |
| 15a | 12. 5 | 12. 5 | 0. 7 | 7. 2 | 0. 7 |

FIG. 11

EP 4 265 172 A1

FIG. 12

| VISUAL TARGET NUMBER | CENTER REGION | INDUCTION REGION | | VISUAL TARGET AVERAGE | BACKGROUND |
|---|---|---|---|---|---|
| | | MAXIMUM VALUE | MINIMUM VALUE | | |
| 1s | 255.0 | 255.0 | 0.7 | 164.0 | 0.7 |
| 2s | 237.2 | 237.2 | 0.7 | 154.5 | 0.7 |
| 3s | 218.8 | 218.8 | 0.7 | 143.5 | 0.7 |
| 4s | 200.5 | 200.5 | 0.7 | 132.0 | 0.7 |
| 5s | 184.1 | 184.1 | 0.7 | 122.1 | 0.7 |
| 6s | 167.3 | 167.3 | 0.7 | 111.3 | 0.7 |
| 7s | 151.3 | 151.3 | 0.7 | 98.7 | 0.7 |
| 8s | 133.5 | 133.5 | 0.7 | 88.9 | 0.7 |
| 9s | 116.0 | 116.0 | 0.7 | 78.7 | 0.7 |
| 10s | 96.0 | 96.0 | 0.7 | 64.8 | 0.7 |
| 11s | 80.5 | 80.5 | 0.7 | 54.8 | 0.7 |
| 12s | 62.7 | 62.7 | 0.7 | 41.3 | 0.7 |
| 13s | 46.1 | 46.1 | 0.7 | 31.8 | 0.7 |
| 14s | 39.1 | 39.1 | 0.7 | 19.0 | 0.7 |
| 15s | 12.5 | 12.5 | 0.7 | 7.8 | 0.7 |

FIG. 13

| VISUAL TARGET NUMBER | CENTER REGION | INDUCTION REGION | | VISUAL TARGET AVERAGE | BACKGROUND |
|---|---|---|---|---|---|
| | | MAXIMUM VALUE | MINIMUM VALUE | | |
| 1b | 255.0 | 255.0 | 128.2 | 222.9 | 0.7 |
| 2b | 237.2 | 237.2 | 118.5 | 199.0 | 0.7 |
| 3b | 218.8 | 218.8 | 107.4 | 188.1 | 0.7 |
| 4b | 200.5 | 200.5 | 103.1 | 162.6 | 0.7 |
| 5b | 184.1 | 184.1 | 91.8 | 158.5 | 0.7 |
| 6b | 167.3 | 167.3 | 85.7 | 143.3 | 0.7 |
| 7b | 151.3 | 151.3 | 75.4 | 129.7 | 0.7 |
| 8b | 133.5 | 133.5 | 68.4 | 114.6 | 0.7 |
| 9b | 116.0 | 116.0 | 57.6 | 99.0 | 0.7 |
| 10b | 96.0 | 96.0 | 50.3 | 82.0 | 0.7 |
| 11b | 80.5 | 80.5 | 39.5 | 67.9 | 0.7 |
| 12b | 62.7 | 62.7 | 32.2 | 53.1 | 0.7 |
| 13b | 46.1 | 46.1 | 22.5 | 38.3 | 0.7 |
| 14b | 39.1 | 39.1 | 15.1 | 24.1 | 0.7 |
| 15b | 12.5 | 12.5 | 6.1 | 9.8 | 0.7 |

FIG. 14

FIXED VIEWPOINT 1000ms

REFERENCE GLARING TARGET 1000ms

BLANK 300ms

GLARING TARGET 1000m

MASK 30s

P61

P62

P63

P64

P65

EP 4 265 172 A1

FIG. 15

| VISUAL TARGET NUMBER | LUMINANCE $(cd/m^2)$ | VALUE RELATIVE TO REFERENCE | AVERAGE EVALUATION VALUE OF SUBJECT (PSYCHOLOGICAL QUANTITY J) |
|---|---|---|---|
| 1a | 255 | 2040 | 12.3 |
| 2a | 237.2 | 1897.6 | 11.3 |
| 3a | 218.8 | 1750.4 | 14.8 |
| 4a | 200.5 | 1604 | 12.0 |
| 5a | 184.1 | 1472.8 | 13.0 |
| 6a | 167.3 | 1338.4 | 9.8 |
| 7a | 151.3 | 1210.4 | 9.7 |
| 8a | 133.5 | 1068 | 9.8 |
| 9a | 116 | 928 | 11.0 |
| 10a | 96 | 768 | 10.6 |
| 11a | 80.5 | 644 | 7.6 |
| 12a | 62.7 | 501.6 | 8.8 |
| 13a | 46.1 | 368.8 | 6.1 |
| 14a | 39.1 | 312.8 | 5.1 |
| 15a | 12.5 | 100 | 4.9 |

FIG. 16

EVALUATION VALUE OF BRIGHTNESS FOR VISUAL TARGET (1a)
IN MAGNITUDE ESTIMATION METHOD

AVERAGE EVALUATION VALUE

RELATIVE VALUE OF LUMINANCE OF CENTER REGION OF VISUAL TARGET

EP 4 265 172 A1

FIG. 17

EVALUATION VALUE OF BRIGHTNESS FOR VISUAL TARGET (1a)
IN MAGNITUDE ESTIMATION METHOD (BOTH LOGARITHMS)

$y = 0.7928x^{0.3663}$

$R^2 = 0.8388$

LOGARITHM OF EVALUATION VALUE OF BRIGHTNESS

LOGARITHM OF LUMINANCE OF CENTER REGION OF VISUAL TARGET $(cd/m^2)$

FIG. 18

```
                    ┌──────────┐
                    │  START   │
                    └────┬─────┘
                         │ ◄─────────────────────┐
                         ▼                        │
        ┌────────────────────────────────┐       │
        │ PRESENT GLARE ILLUSION IMAGE    │─ S10  │
        └────────────────┬───────────────┘       │
                         ▼                        │
        ┌────────────────────────────────┐       │
        │       RECEIVE OPERATION         │─ S20  │
        └────────────────┬───────────────┘       │
                         ▼                        │
        ┌────────────────────────────────┐       │
        │  ACQUIRE RESPONSE INFORMATION   │─ S30  │
        └────────────────┬───────────────┘       │
                         ▼        S40             │
                      ╱──────────╲               │
                    ╱   HAS END    ╲     NO       │
                   ⟨ CONDITION BEEN  ⟩────────────┘
                    ╲  SATISFIED?  ╱
                      ╲──────────╱
                         │ YES
                         ▼
        ┌────────────────────────────────┐
        │     CALCULATE GLARE INDEX       │─ S50
        └────────────────┬───────────────┘
                         ▼
        ┌────────────────────────────────┐
        │      OUTPUT GLARE INDEX         │─ S60
        └────────────────┬───────────────┘
                         ▼
                    ┌──────────┐
                    │   END    │
                    └──────────┘
```

# FIG. 19

FIG. 20

FIG. 20

1d

VISUAL FUNCTION EXAMINATION DEVICE — 2d

CONTROL UNIT — 20d

A1; GLARE ILLUSION IMAGE

8(3)

OUTPUT DEVICE

A3; GLARE INDEX

GLARE ILLUSION IMAGE PRESENTATION UNIT — 200

PARAMETER ACQUISITION UNIT — 205d

OPERATION RECEPTION UNIT — 201d

GLARE ILLUSION IMAGE GENERATION UNIT — 206d

RESPONSE INFORMATION ACQUISITION UNIT — 202

A2; RESPONSE INFORMATION

4d

INPUT DEVICE

A4; PARAMETER

GLARE INDEX CALCULATION UNIT — 203

PRESENTATION UNIT — 204

STORAGE UNIT — 210 — 21

GLARE ILLUSION IMAGE INFORMATION

EP 4 265 172 A1

FIG. 21

A5: SPECTACLE LENS INFORMATION

1e

A1: GLARE ILLUSION IMAGE

8(3)

OUTPUT DEVICE

A3: GLARE INDEX

A2: RESPONSE INFORMATION

4

INPUT DEVICE

VISUAL FUNCTION EXAMINATION DEVICE — 2

CONTROL UNIT — 20

GLARE ILLUSION IMAGE PRESENTATION UNIT — 200

OPERATION RECEPTION UNIT — 201

RESPONSE INFORMATION ACQUISITION UNIT — 202

GLARE INDEX CALCULATION UNIT — 203

PRESENTATION UNIT — 204

STORAGE UNIT — 210
21 —
GLARE ILLUSION IMAGE INFORMATION

A3: GLARE INDEX

SPECTACLE LENS PRESENTATION DEVICE — 9e

CONTROL UNIT — 90e

SPECTACLE LENS DECISION UNIT — 900e

SPECTACLE LENS INFORMATION PRESENTATION UNIT — 901e

STORAGE UNIT — 91e

EP 4 265 172 A1

FIG. 22

A5;SPECTACLE LENS INFORMATION, A6;SPECTACLE LENS WEARING IMAGE

1f

A1;GLARE ILLUSION IMAGE

8(3)

OUTPUT DEVICE

A3;GLARE INDEX

A2;RESPONSE INFORMATION

4

INPUT DEVICE

VISUAL FUNCTION EXAMINATION DEVICE — 2

CONTROL UNIT — 20

GLARE ILLUSION IMAGE PRESENTATION UNIT — 200

OPERATION RECEPTION UNIT — 201

RESPONSE INFORMATION ACQUISITION UNIT — 202

GLARE INDEX CALCULATION UNIT — 203

PRESENTATION UNIT — 204

21 — STORAGE UNIT — 210

GLARE ILLUSION IMAGE INFORMATION

A3;GLARE INDEX

A1;GLARE ILLUSION IMAGE

SPECTACLE LENS PRESENTATION DEVICE — 9f

CONTROL UNIT — 90f

SPECTACLE LENS DECISION UNIT — 900e

SPECTACLE LENS INFORMATION PRESENTATION UNIT — 901e

SPECTACLE LENS WEARING IMAGE GENERATION UNIT — 902f

SPECTACLE LENS WEARING IMAGE PRESENTATION UNIT — 903f

STORAGE UNIT — 91e

EP 4 265 172 A1

FIG. 23

（A）

（B）

P71

P72

EP 4 265 172 A1

FIG. 24

| ORIGINAL VISUAL TARGET (NO SPECTACLE LENSES) | | VISUAL TARGET (15% TRANSMITTANCE CUT) | | VISUAL TARGET (25% TRANSMITTANCE CUT) | | VISUAL TARGET (35% TRANSMITTANCE CUT) | |
|---|---|---|---|---|---|---|---|
| VISUAL TARGET NUMBER | VISUAL TARGET AVERAGE | VISUAL TARGET NUMBER | VISUAL TARGET AVERAGE | VISUAL TARGET NUMBER | VISUAL TARGET AVERAGE | VISUAL TARGET NUMBER | VISUAL TARGET AVERAGE |
| 1a | 159. 3 | 1a-15 | 135. 4 | 1a-25 | 111. 3 | 1a-35 | 94. 3 |
| 2a | 146. 6 | 2a-15 | 124. 6 | 2a-25 | 102. 8 | 2a-35 | 87. 2 |
| 3a | 134. 5 | 3a-15 | 114. 3 | 3a-25 | 93. 2 | 3-35 | 79. 3 |
| 4a | 122. 9 | 4a-15 | 104. 5 | 4a-25 | 86. 2 | 4a-35 | 73. 5 |
| 5a | 112. 9 | 5a-15 | 96. 0 | 5a-25 | 79. 1 | 5a-35 | 67. 7 |
| 6a | 102. 1 | 6a-15 | 86. 8 | 6a-25 | 71. 8 | 6a-35 | 61. 5 |
| 7a | 92. 2 | 7a-15 | 78. 4 | 7a-25 | 64. 8 | 7a-35 | 55. 7 |
| 8a | 80. 9 | 8a-15 | 68. 8 | 8a-25 | 56. 9 | 8a-35 | 48. 8 |
| 9a | 70. 3 | 9a-15 | 59. 8 | 9a-25 | 49. 5 | 9a-35 | 42. 4 |
| 10a | 58. 0 | 10a-15 | 49. 3 | 10a-25 | 40. 9 | 10a-35 | 35. 1 |
| 11a | 48. 3 | 11a-15 | 41. 1 | 11a-25 | 34. 5 | 11a-35 | 29. 6 |
| 12a | 37. 6 | 12a-15 | 32. 0 | 12a-25 | 26. 8 | 12a-35 | 23. 0 |
| 13a | 27. 4 | 13a-15 | 23. 3 | 13a-25 | 19. 6 | 13a-35 | 16. 8 |
| 14a | 17. 2 | 14a-15 | 14. 6 | 14a-25 | 12. 3 | 14a-35 | 10. 5 |
| 15a | 7. 2 | 15a-15 | 6. 1 | 15a-25 | 5. 2 | 15a-35 | 4. 4 |

FIG. 25

| VISUAL TARGET (50% TRANSMITTANCE CUT) | | VISUAL TARGET (75% TRANSMITTANCE CUT) | | VISUAL TARGET (85% TRANSMITTANCE CUT) | |
|---|---|---|---|---|---|
| VISUAL TARGET NUMBER | VISUAL TARGET AVERAGE | VISUAL TARGET NUMBER | VISUAL TARGET AVERAGE | VISUAL TARGET NUMBER | VISUAL TARGET AVERAGE |
| 1a-50 | 79.6 | 1a-75 | 40.8 | 1a-85 | 24.4 |
| 2a-50 | 73.7 | 2a-75 | 37.7 | 2a-85 | 22.6 |
| 3a-50 | 66.9 | 3a-75 | 34.2 | 3a-85 | 20.6 |
| 4a-50 | 62.2 | 4a-75 | 31.6 | 4a-85 | 19.2 |
| 5a-50 | 57.3 | 5a-75 | 29.0 | 5a-85 | 17.7 |
| 6a-50 | 51.8 | 6a-75 | 26.3 | 6a-85 | 16.0 |
| 7a-50 | 46.9 | 7a-75 | 23.8 | 7a-85 | 14.5 |
| 8a-50 | 41.3 | 8a-75 | 20.9 | 8a-85 | 12.8 |
| 9a-50 | 36.0 | 9a-75 | 18.2 | 9a-85 | 11.2 |
| 10a-50 | 28.0 | 10a-75 | 15.1 | 10a-85 | 9.3 |
| 11a-50 | 23.8 | 11a-75 | 12.7 | 11a-85 | 7.8 |
| 12a-50 | 16.2 | 12a-75 | 9.9 | 12a-85 | 6.1 |
| 13a-50 | 11.4 | 13a-75 | 7.2 | 13a-85 | 4.5 |
| 14a-50 | 9.0 | 14a-75 | 4.6 | 14a-85 | 2.8 |
| 15a-50 | 3.8 | 15a-75 | 2.0 | 15a-85 | 1.2 |

FIG. 26  1g

VISUAL FUNCTION
EXAMINATION DEVICE — 2g

CONTROL UNIT — 20b

RESPONSE INFORMATION
ACQUISITION UNIT — 202

GLARE INDEX
CALCULATION UNIT — 203

STORAGE UNIT — 21b

COMMUNICATION UNIT — 22b

B1

4g

A2 ; RESPONSE
INFORMATION

A3 ; GLARE INDEX

T1

EP 4 265 172 A1

## INTERNATIONAL SEARCH REPORT

International application No.

**PCT/JP2021/043584**

### A. CLASSIFICATION OF SUBJECT MATTER

***A61B 3/06***(2006.01)i
FI:   A61B3/06

According to International Patent Classification (IPC) or to both national classification and IPC

### B. FIELDS SEARCHED

Minimum documentation searched (classification system followed by classification symbols)

A61B3/06

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); PubMed

### C. DOCUMENTS CONSIDERED TO BE RELEVANT

| Category* | Citation of document, with indication, where appropriate, of the relevant passages | Relevant to claim No. |
|---|---|---|
| X | FACCHIN, Alessio et al., The Glare Effect Test and the Impact of Age on Luminosity Thresholds, frontiers in Psychology, 30 June 2017, vol. 8, no. 1132, pp. 1-5, <PMID: 28713326>,<DOI: 10.3389/fpsyg.2017.01132><br>    fig. 1 | 7 |
| A | | 1-6, 8-10 |
| A | SUZUKI, Yuta et al., Colorful glares: Effects of colors on brightness illusions measured with pupillometry, Acta Psychologica, 2019, vol. 198, Article 102882<br>    fig. 1, 2 | 1-10 |
| A | JP 2020-130581 A (AISIN SEIKI CO., LTD.) 31 August 2020 (2020-08-31)<br>    abstract, paragraphs [0033]-[0042], fig. 1, 2 | 1-10 |
| A | JP 2015-211719 A (TOPCON CORP.) 26 November 2015 (2015-11-26)<br>    abstract, paragraphs [0018]-[0032], fig. 1 | 1-10 |
| A | JP 2007-68925 A (REFRACTIVE EYE CLINIC MEDICAL CORP.) 22 March 2007 (2007-03-22)<br>    paragraphs [0002]-[0004] | 1-10 |

☑ Further documents are listed in the continuation of Box C.    ☑ See patent family annex.

| | |
|---|---|
| * Special categories of cited documents:<br>"A"  document defining the general state of the art which is not considered to be of particular relevance<br>"E"  earlier application or patent but published on or after the international filing date<br>"L"  document which may throw doubts on priority claim(s) or which is cited to establish the publication date of another citation or other special reason (as specified)<br>"O"  document referring to an oral disclosure, use, exhibition or other means<br>"P"  document published prior to the international filing date but later than the priority date claimed | "T"  later document published after the international filing date or priority date and not in conflict with the application but cited to understand the principle or theory underlying the invention<br>"X"  document of particular relevance; the claimed invention cannot be considered novel or cannot be considered to involve an inventive step when the document is taken alone<br>"Y"  document of particular relevance; the claimed invention cannot be considered to involve an inventive step when the document is combined with one or more other such documents, such combination being obvious to a person skilled in the art<br>"&"  document member of the same patent family |

| Date of the actual completion of the international search | Date of mailing of the international search report |
|---|---|
| **26 January 2022** | **08 February 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**

| International application No. |
| --- |
| **PCT/JP2021/043584** |

**C. DOCUMENTS CONSIDERED TO BE RELEVANT**

| Category* | Citation of document, with indication, where appropriate, of the relevant passages | Relevant to claim No. |
| --- | --- | --- |
| A | JP 2008-93131 A (TOYOTA MOTOR CORP.) 24 April 2008 (2008-04-24)<br>abstract | 1-10 |
| A | JP 2019-209047 A (MITSUI CHEMICALS, INC.) 12 December 2019 (2019-12-12)<br>abstract | 1-10 |
| A | WO 2019/155025 A1 (AMO GRONINGEN B.V.) 15 August 2019 (2019-08-15)<br>paragraphs [0015]-[0050], fig. 1-5 | 1-10 |

Form PCT/ISA/210 (second sheet) (January 2015)

**INTERNATIONAL SEARCH REPORT**
**Information on patent family members**

International application No.

**PCT/JP2021/043584**

| Patent document cited in search report | | | Publication date (day/month/year) | Patent family member(s) | | Publication date (day/month/year) |
|---|---|---|---|---|---|---|
| JP | 2020-130581 | A | 31 August 2020 | (Family: none) | | |
| JP | 2015-211719 | A | 26 November 2015 | (Family: none) | | |
| JP | 2007-68925 | A | 22 March 2007 | (Family: none) | | |
| JP | 2008-93131 | A | 24 April 2008 | (Family: none) | | |
| JP | 2019-209047 | A | 12 December 2019 | (Family: none) | | |
| WO | 2019/155025 | A1 | 15 August 2019 | US | 2019/0239803 A1 | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2020211755 A
- JP 2008093131 A